Europäisches Patentamt
European Patent Office
Office européen des brevets

(11) Publication number: 0 414 247 A2

(12) EUROPEAN PATENT APPLICATION

(21) Application number: 90116180.2

(22) Date of filing: 23.08.90

(51) Int. Cl.5: C12N 15/55, C12N 15/62, C12N 15/70, C12P 7/40, C12N 9/18, C12P 41/00, //(C12N9/18,C12R1:38)

The applicant has subsequently filed a sequence listing and declared, that it includes no new matter.

(30) Priority: 24.08.89 US 398102
07.08.90 US 562491

(43) Date of publication of application: 27.02.91 Bulletin 91/09

(84) Designated Contracting States: AT BE CH DE DK ES FR GB GR IT LI LU NL SE

(71) Applicant: SYNTEX PHARMACEUTICALS INTERNATIONAL LIMITED
Corner House Church Street
Hamilton 5(BM)

(72) Inventor: Chan, Hardy W.
2755 St. James Road
Belmont, CA 94002(US)
Inventor: Salazar, Felix H.
706 Barron Avenue
Palo Alto, CA 94306(US)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40(DE)

(54) **Cloning, expression and sequencing of an ester hydrolase genein escherichia coli.**

(57) The invention relates to the construction of recombinant DNA molecules containing an ester hydrolase gene from Pseudomonas fluorescens and the generation of the cloned hydrolase and mutants thereof in Escherichia coli . The cloned hydrolases produced are partioularly useful in the production of S-naproxen by enzymatic hydrolysis of R,S-naproxen esters in an enantiospecific fashion.

EP 0 414 247 A2

# CLONING, EXPRESSION AND SEQUENCING OF AN ESTER HYDROLASE GENE IN ESCHERICHIA COLI

This invention relates to the production of an ester hydrolase. More particularly, it relates to the construction of recombinant molecules containing an ester hydrolase gene from Pseudomonas fluorescens and the generation of the cloned hydrolase and mutants thereof in Escherichia coli . The cloned hydrolases produced in such a manner were found to be especially useful in a process in which R-,S-naproxen esters were enzymatically hydrolyzed in an enantiospecific fashion.

A number of microorganisms, as well as a few selected commercial lipases and esterases, have been reported to hydrolyze naproxen esters with some chiral preference (Iriuchijima and Keiyu, Agr. Biol. Chem . 45 :1389 (1981); Gu, et al ., Tetrahedron Lett. 27 :1763 (1986)). The actual application of these microorganisms or enzymes in naproxen manufacturing, however, has been made impractical by their lack of absolute chiral specificity and the relative low rates of conversion. For example, an overnight culture of Pseudomonas fluorescens gave extremely low conversion of racemic naproxen ester into S-naproxen and the enantiomeric excess (ee) of the final product was found to be less than 96%.(European Patent Application EP 0 233 656). Likewise, Candida cylindracea lipase, a commercially available enzyme, at 22 °C required several days to convert racemic naproxen methyl ester into S-naproxen (European Patent Application EP 0 227 078).

European Patent Application EP 0 195 717 describes the use of commercially available lipase from Candida cylindracea which results in an optically active alpha-arylalkanoic acid produced in essentially the S-form. Using the described method, the final processing step requires a separation of the enantiomeric forms in order to obtain the S-form of the compound. When used in the production of naproxen the conversion of racemic ester was reported as being approximately 32%.

European Patent Application EP 0 233 656 describes a process for the preparation of 2-arylpropionic acids in which the use of a microorganism having the ability to stereoselectively hydrolyze a racemic mixture of the lower alkyl esters results in the formation of an acid having at least 80% by weight of the S-configuration.

EP 0 227 078 describes the process of preparing S-alpha-methylarylacetic acids from mixtures using extracellular lipases of microbial origin.

Nakagawa, et.al . J. Biochem 95 :1047 (1984) describe an intracellular esterase isolated from Pseudomonas fluorescens which catalizes the hydrolysis of methyl esters of short chain length. The intracellular esterase differs from known extracellular lipases in its sensitivity to inhibitors, molecular weight and substrate specificity.

Elevation of temperature, in general, accelerates the rate of enzymatic reaction. In the case of R-,S-naproxen ester hydrolysis, it may also convert the solid ester substrate into its liquid form. It is, therefore, desirable to conduct the hydrolysis at the highest temperature which could be tolerated by the ester hydrolase. To this end, it is desirable to develop an ester hydrolase which would exhibit a high thermal stability in the hydrolysis process.

One embodiment of the invention is a DNA molecule comprising a gene for ester hydrolase obtained from Pseudomonas fluorescens and encoded within a 2.4 kb Hind III fragment of the DNA molecule.

Another embodiment of the invention is a gene which encodes the amino acid sequence for authentic ester hydrolase.

Another embodiment of the invention is a gene which encodes the amino acid sequence for a fused ester hydrolase.

Another embodiment of the invention is a DNA molecule comprising a gene for ester hydrolase obtained from a microbial host selected from the group Pseudomonas fluorescens , Pseudomonas mendocina and Pseudomonas stutzeri .

Another embodiment of the invention is a DNA molecule comprising a gene for a cloned ester hydrolase obtained from Pseudomonas fluorescens encoded within a 2.4 kb Hind III fragment of the DNA molecule or temperature resistant mutants thereof placed downstream to regulatory elements functional in E. col i.

Another embodiment of the invention is a process for the production of naproxen wherein a cloned ester hydrolase is used to enantioselectively catalyze the hydrolysis of an R,S-naproxen alkyl ester. The reaction comprises the steps of (1) producing in E. coli a cloned ester hydrolase from Pseudomonas fluorescens having improved performance characteristics; (2) presenting the cloned ester hydrolase to the naproxen ester mixture either as free soluble enzyme or in its immobilized form; (3) hydrolyzing the R-,S-naproxen ester; and (4) collecting S-naproxen.

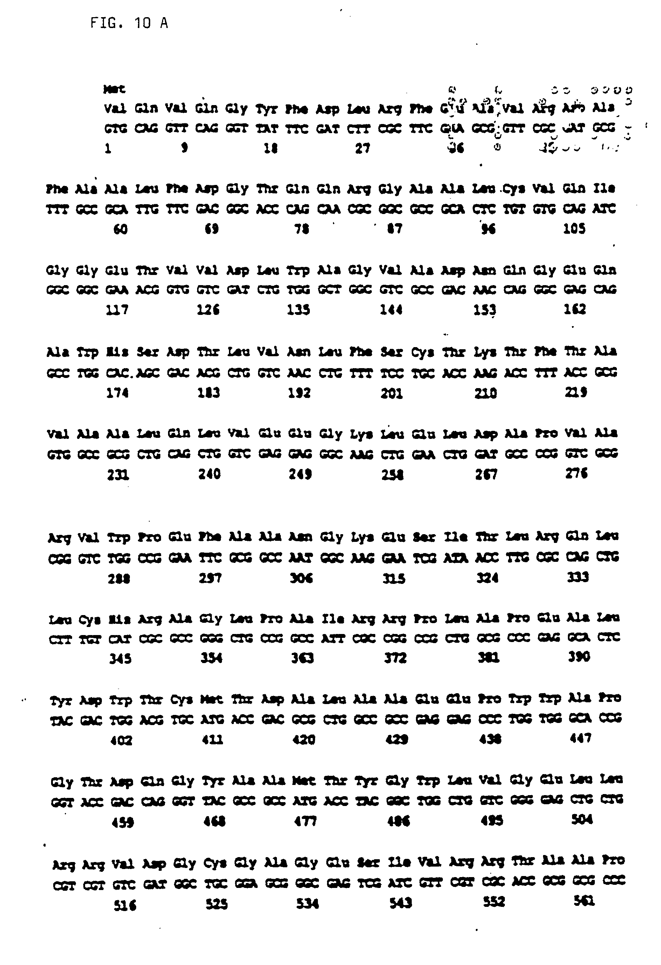

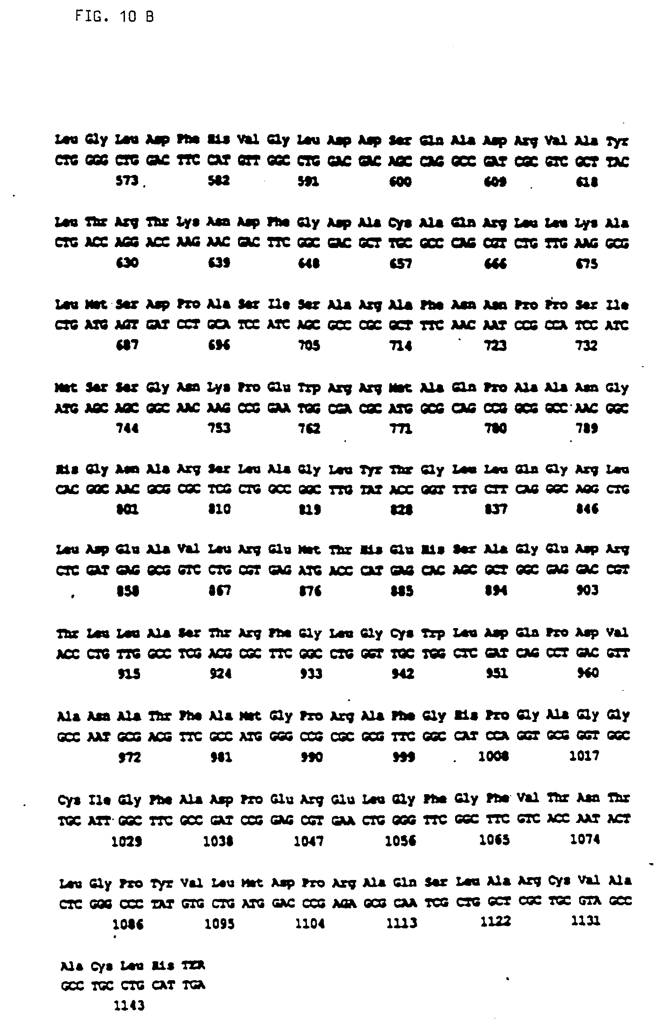

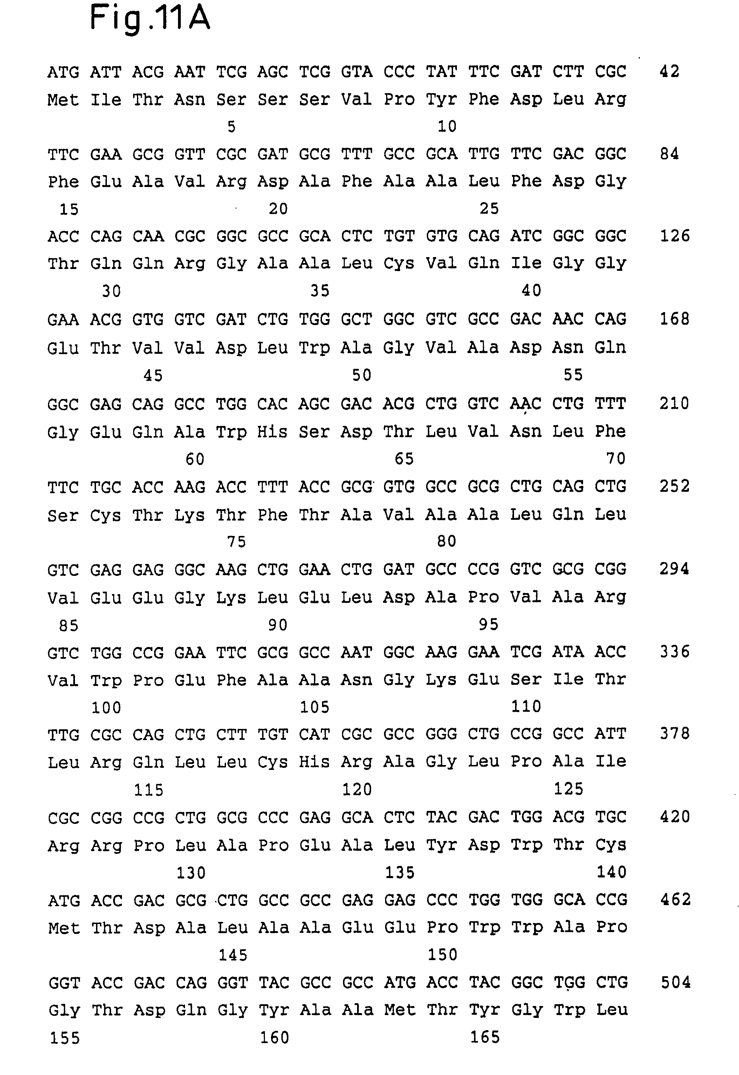

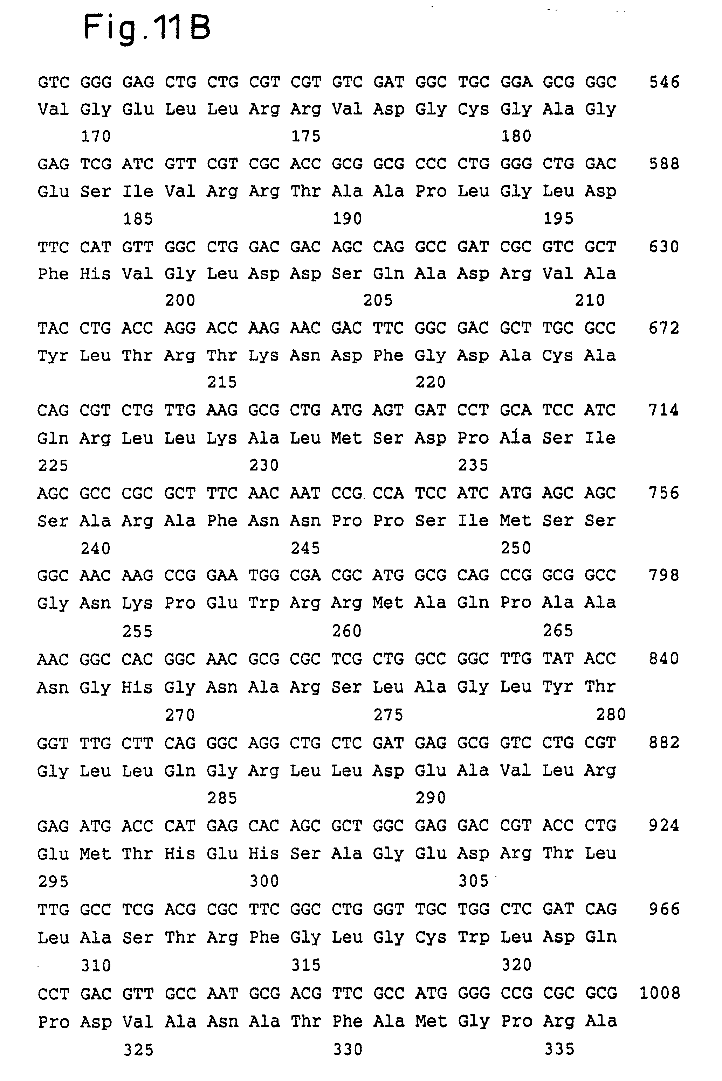

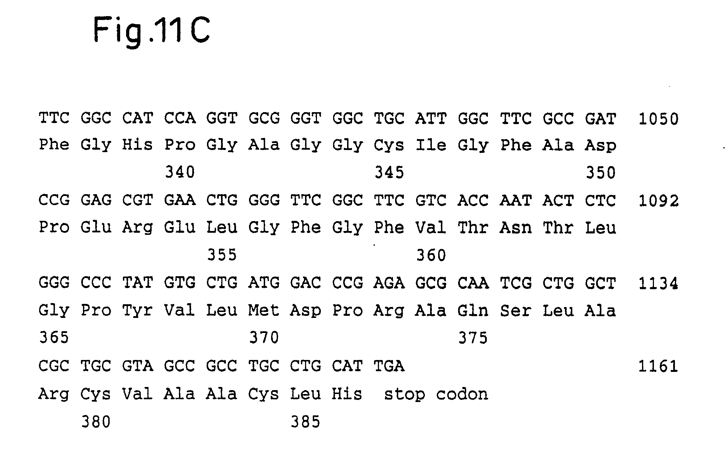

Another embodiment of the invention is an ester hydrolase having the amino acid sequence as set forth in Figure 10 or Figure 11, or a sequence related thereto by substitution, addition or deletion of one or more amino acids and capable of effecting the hydrolysis of R,S naproxen esters to S-naproxen in an enantiomeric excess (ee) of greater than 97%.

Another embodiment of the invention is the process for the production of S-naproxen or a pharmaceutically acceptable salt thereof which comprises subjecting an R,Snaproxen ester to the action of a cloned ester hydrolase to afford chirally specific S-naproxen.

## Figures

Figure 1 Activity staining of the cloned ester hydrolase resolved on a non-denaturing gel
Figure 2 Thermal stability of P. fluorescens ester hydrolase
Figure 3 Effect of detergent and purity on activity of ester hydrolase
Figure 4 Amino Terminal Sequence of P. fluorescens ester hydrolase
Figure 5 Plasmid map pPF-3A
Figure 6 Hydrolysis of ester hydrolase showing preference for S-naproxen ester
Figure 7 Nucleotide and amino acid sequence at the lac Z-ester hydrolase fusion junction in pPF-GD3A
Figure 8 Thermal stability of esterase mutants at 60 °C temperature
Figure 9 Comparison of Amino Terminal Sequence of P. fluorescens ester hydrolase in PF-3A and as a fusion protein in pPF-GD3A
Figure 10 Complete nucleotide and amino acid sequence of authentic ester hydrolase
Figure 11 Complete nucleotide and amino acid sequence of the fused ester hydrolase

The present invention is a means to produce an ester hydrolase. Using Pseudomonas fluorescens , an ester hydrolase gene was transformed into Escherichia coli . The cloned ester hydrolase enzyme expressed by the transformed E. coli , is distinctly different from enzymes previously decribed by Sugiura, Biochim. Biophys. Acta 488 :353 (1977), ibid, 489 :262 (1977) and Nakagawa, J. Biochem. 95 :1047 (1984). The resulting expressed cloned ester hydrolase was found to exhibit high enantiospecificity for the esters of S-naproxen. A mutated form of the ester hydrolase was also generated which showed improved thermal stability with similar enantiospecificity.

Before proceeding further, the following terms shall be defined:

"Cloned ester hydrolase" shall mean an ester hydrolase enzyme obtained by the cloning of the gene for ester hydrolase into a suitable expression system. A cloned ester hydrolase may be an "authentic ester hydrolase", a "fused ester hydrolase" or a "temperature-resistant ester hydrolase". Whether used in the singular or plural form, the cloned ester hydrolase shall refer to these particularly defined enzymes, either as a group or individually.

"Authentic ester hydrolase" shall mean the cloned ester hydrolase having the complete nucleotide sequence obtained from the genome of P. fluorescens with an N-terminal methionine, which may be encoded by a codon normally used for valine, and having no deletions or mutations.

"Fused ester hydrolase" shall mean the cloned ester hydrolase having a sequence expressed as a fusion between all or a portion of the authentic sequence for ester hydrolase and a heterologous protein.

"Temperature-resistant ester hydrolase" shall mean a cloned mutation of the authentic ester hydrolase obtained by random or site-directed mutagenesis wherein the mutant form of the ester hydrolase is resistant to high temperatures. The temperature-resistant ester hydrolase has at least a 75% amino acid sequence homology with the authentic ester hydrolase.

"Derivative" of the cloned ester hydrolase shall mean a cloned ester hydrolase having an amino acid sequence which differs from the authentic ester hydrolase by amino acid additions, deletions or substitutions in a manner such that the resulting derivative has at least a 75% amino acid sequence homology with the authentic ester hydrolase and exhibits essentially the same activity as the authentic ester hydrolase.

"Regulatory region" shall mean the expression control sequence, for example, a promoter and ribosome binding site, necessary for transcription and translation.

The cloned ester hydrolase has the capability to convert R,S-naproxen esters to S-naproxen. The ester hydrolase has a characteristic N-terminal amino acid sequence responsible for the enantioselective hydrolysis to S-naproxen.

The term "R,S-naproxen ester" shall mean a mixture of R- and S-enantiomers of varying ratios of an ester of 2-(6′-methoxy-2′-naphthy)propionic acid. This term shall also include the esters of naproxen to naproxen precursors convertible to naproxen.

Naproxen is a known anti-inflammatory drug having the chemical name 2-(6′-methoxy-2′-naphthyl)

propionic acid which can exist as two optical isomers. Naproxen is the S-enantiomer according to the Cahn-Ingold-Prelog rule of asymmetric configuration and is recognized to be substantially more biologically active than its optical counterpart, the R-enantiomer.

The ester portion of the R,S-naproxen ester generally comprises a straight, branched, or cyclic alkyl group having from 1 to 12 carbon atoms, optionally substituted with phenyl or one or more electron-withdrawing substituents, for example halo, nitro, cyano, hydroxy, $C_{1-4}$ alkoxy, $C_{1-4}$ alkylthio, or -C(O)$R^1$ wherein $R^1$ is $C_{1-4}$ alkyl, $C_{3-6}$ cycloalkyl, hydroxy, $C_{1-4}$ alkoxy, $C_{3-6}$ cycloalkoxy, phenoxy, benzyloxy, $NR^2R^3$ (in which $R^2$ and $R^3$ are independently H, $C_{1-4}$ alkyl, $C_{3-6}$ cycloalkyl, or jointly form a 5- or 6-membered ring together with the nitrogen, the ring optionally including a hetero group selected from O, NH, or N-($C_{1-4}$ alkyl), or -OM wherein M is an alkali metal.

The electron-withdrawing substituents if present are preferably at the $\alpha$- or $\beta$- position of the R group, to the extent consistent with the stability of the group. Esters in which the R groups contain electron-withdrawing substituents are referred to as activated esters, since they generally hydrolyze more rapidly than those where the R group is not so substituted.

Specific examples of alkyl groups, R, are methyl, ethyl, butyl, hexyl, octyl, dodecyl, benzyl, 2-chloroethyl, 2,2,2-trichloroethyl, 2-fluoroethyl, 2,2,2-trifluoroethyl, 2-bromoethyl, cyanomethyl, 2-nitropropyl, carboethoxymethyl, methoxymethyl, 2 -hydroxy- 1 , 2 -dimethoxycarbonylethyl, 2 -hydroxy-1, 2 -dicarboxyethyl, 2-hydroxy-1,2-diethoxycarbonylethyl, and the like.

If the S-acid resulting from the enantioselective hydrolysis is a precursor of naproxen, for example ( S )-5-halo-6-methoxy-$\alpha$-methyl-2-naphthaleneacetic acid, ( S )-6-hydroxy-$\alpha$-methyl-2-naphthaleneacetic acid or ( S )-5-halo-6-hydroxy-$\alpha$-methyl-2-naphthaleneacetic acid, such precursor can be converted to naproxen by methods described in European Published Application 95901 (1983). It has previously been reported, (see European patent Application EP 0 233 656) that a broad range of microorganisms belonging to the genera Bacillus, Pseudomonas , Arthrobacter , Mucor and Streptomyces , have the ability to stereoselectively hydrolyze R-,S-naproxen esters to form S-naproxen.

An alternative to using whole or disrupted microorganisms is to employ microbial enzymes derived from the whole microorganism. However, such a method has given equivocal results. For example, commercial enzyme preparations from Pseudomonas fluorescens were unable to efficiently hydrolyze R-,S-naproxen esters in an enantiospecific manner. This underscores the fact that most microorganisms contain multiple genes which code for a variety of lipases or esterases with only a subset of these having the desired specificity of use in a particular application. Hence, the ability to identify and isolate an enzyme from any microorganism having the desired characteristics, regardless of the microorganism's substrate preference, is not predictable. One, in fact, needs to set out to search for the enzyme exhibiting the desired characteristics and purify it in quantity, before one can evaluate its utility in an actual industrial context. Often, the desired enzyme requires additional chemical or genetic modifications to render its practical utility in a production process. This application describes novel cloned ester hydrolases from the gram negative bacterium, Pseudomonas fluorescens . The cloned ester hydrolases were found to be different from the known lipases and esterases from the same species, both in terms of structure and enzymatic properties. Related ester hydrolases have also been cloned from members of the same genus, which include, for example, Pseudomonas mendocina and Pseudomonas stutzeri . In a preferred practice of the invention, the cloned enzyme, which has a long half life at approximately 45° C, was further mutagenized to generate an enzyme of improved thermal stability. The cloned enzyme upon mutagenesis has a stability preferably from 45-65° C, most preferably 62° C.

In one practice of the invention, the molecular cloning of the Pseudomonas fluorescens (hereinafter P. fluorescens ) ester hydrolase gene was carried out using standard molecular cloning techniques (see, for example, Maniatis, et.al. Molecular Cloning: A Laboratory Manual , Second Edition, Cold Spring Harbor Laboratory Press (1989)). DNA was prepared from P. fluorescens (ATCC #17550) and partially cleaved with restriction enzymes. The partial DNA digest was ligated with enzyme-cleaved DNA suitable for use as a cloning plasmid, for example pUC19 and the like, and transformed into an E. coli strain devoid of detectable basal esterase activity.

While other host organisms such as B. subtilis may be used, the E. coli bacterium is the organism of choice for the production of the cloned enzyme. Cloning and expression can be obtained rapidly in E. coli and high levels of gene expression are common. In addition, production in E. coli results in a system easily scaled up for large scale fermentation and protein purification.

Initial in situ screens for esterolytic activity employed beta-naphthyl acetate as a substrate. When R-,S-naproxen esters were used as the substrate, a subset of the esterase positive clones from the various Pseudomonas species showed activity and evidenced enantiospecificity. In particular, when the S- or R-isomer of naproxen ester was given to the ester hydrolase positive clones, a number of the clones showed strong preference for the S-naproxen ester. DNA cross hybridization experiments showed the ester hydrolase genes to be highly conserved among different Pseudomonas species.

In one practice of the invention, positive ester hydrolase clones from P. fluorescens identified using the procedures described in Example 1 were chosen for further characterization and study. Plasmid DNA from one such identified clone was prepared using standard techniques. The DNA insert was found to be 4.5 kilobases (kb) in length. Subcloning experiments as described in Example 2 indicated that the esterase gene was encoded within a 2.4 kb Hind III fragment.

The experiments also suggested that the expression of the P. fluorescens ester hydrolase gene in E. coli was driven by the E. coli promoter ( lac P-O) of the lac operon rather than its own endogenous promoter. When the 2.4 kb Hind III fragment was purified and recloned into cloning plasmids, for example, pUC18 and pUC19 (Yanisch-Perron, et al ., Gene 33 :103 (1985)), all the esterase positive clones had the DNA inserted in the same orientation with respect to transcriptional initiation from the lac promoter of pUC. These results suggested that either the 2.4 kb P. fluorescens DNA was devoid of a Pseudomonas promoter, or if present, the endogenous promoter was not functional in E. coli . Further studies indicated that the promoter being used was in fact an E. coli promoter. Other E. coli promoters which may be used in the practice of the invention include, for example, trp , tac , lambda $P_L$ and lambda $P_R$.

Further characterization of the P. fluorescen s cloned ester hydrolase involved a study of the effect of isopropylthio-galactoside (IPTG) on the expression of the ester hydrolase. IPTG is known to induce transcription of the lactose ( lac ) operon in E. coli . Other induction schemes can be used in the practice of the invention and will be dependent upon the promoter being used in the expression system.

Induction of a bacterial culture of the invention were significantly induced with IPTG. A 3-5 fold increase in esterolytic activity was seen when the culture was induced with IPTG. When protein extracts from the control and induced cultures were analyzed on SDS-polyacrylamide gels (that is, in a denatured system), a protein of approximately 33 kilodalton (kD) was observed. Subsequent studies which included nucleotide and amino acid sequencing of the ester hydrolase gene and its protein product suggest that the protein has a calculated molecular weight closer to 42 kD. Such variation in molecular weight determination is not uncommon when physical measurements are used as the primary means for determination ( Methods in Enzymology , Vol. 104, Part C, W.B. Jakoby, Editor).

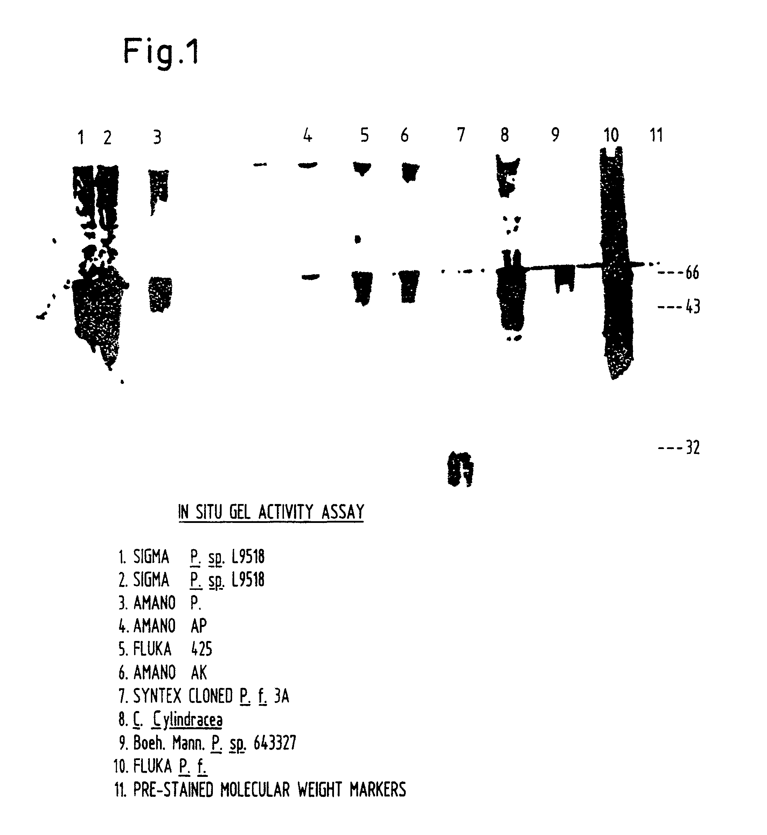

Activity staining of the cloned ester hydrolase and other commercially available Pseudomonas lipases in a non-denaturing system verified the hydrolase activity of the cloned ester hydrolase while indicating that the cloned ester hydrolase of the invention was distinctly different from commercial Pseudomonas enzymes (Figure 1). That is, initial experiments using a non-denaturing gel system showed the cloned ester hydrolase enzyme having a migration pattern in the non-denaturing system diferent from other known commercial Pseudomonas lipases (Figure 1).

Commercially available enzymes are limited in the extent to which they can be used at elevated temperatures. Use of an cloned ester hydrolase which exhibited high thermal stability was especially desirable in the process for hydrolyzing the naproxen ester.

The economics of an enzyme-catalyzed chemical reaction, be that a continuous or batch process, depends greatly on the lifetime of the catalytically active enzyme. Since thermal inactivation is the most common cause of enzyme inactivation, an increase in thermal stability acts to prolong the life span of the biocatalyst which in turn improves the economics of the overall process.

In addition, the rate of an enzymatic reaction depends on the reaction temperature. An enzyme exhibiting thermal stability permits running the reaction at a higher temperature which accelerates the rate which in turn increases the production through-put. In the case of R-,S-naproxen ester hydrolysis, high temperature also drives the solid ester substrate towards its molten form, rendering the control of solid particle size less critical.

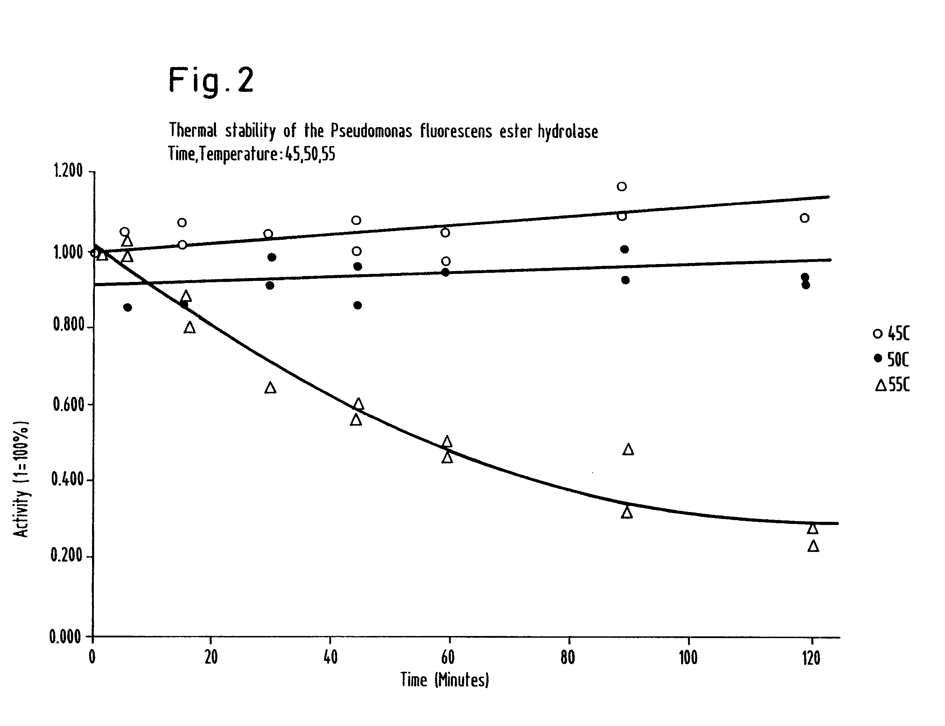

The thermal stability of the cloned P. fluorescens ester hydrolase of the invention was determined by incubating the authentic ester hydrolase at 5 degree intervals between 45° C and 55° C, and subsequently assaying for residual activity. Figure 2 shows the half life of the authentic ester hydrolase to be approximately 90 minutes at 55° C and greater than 4 hours at 45-50° C. Additional studies indicated the authentic ester hydrolase to be active in a range from 30° C to 60° C, preferably 45° C to 55° C. Similar studies with the temperature-resistant ester hydrolase showed the temperature-resistant ester hydrolase to be active in a range from 30° C to 65° C, preferably 45° C to 65° C, most preferably 62° C

In one practice of the invention, the thermal stability of the authentic ester hydrolase was further evaluated. At pH 8.5, the half life of the authentic ester hydrolase at 50° C was found to be at least 6 hours. The enantiospecificity of the authentic ester hydrolase was also evaluated at both 37° C and 50° C, using either a crude or purified preparation of the enzyme and R-,S-naproxen ethyl ester as substrate. Both crude and purified enzyme yielded S-naproxen of high enantiomeric excess (ee>97%) at both temperatures.

Many ester hydrolases, particularly those that are traditionally referred to as esterases, require the addition of detergents or surfactants for optimal activity. Employment of detergents or surfactants in a manufacturing process can be quite costly and its removal requires additional process technology, equipment and labor. Many surfactants can also serve as substrates for the esterase, for example, soy oil or Tween-80 are substrates for some esterases. Hydrolysis of surfactant can also result in the introduction of undesirable contaminants. Hence, ability to run the esterolytic reaction without the need for surfactants is a highly desirable characteristic.

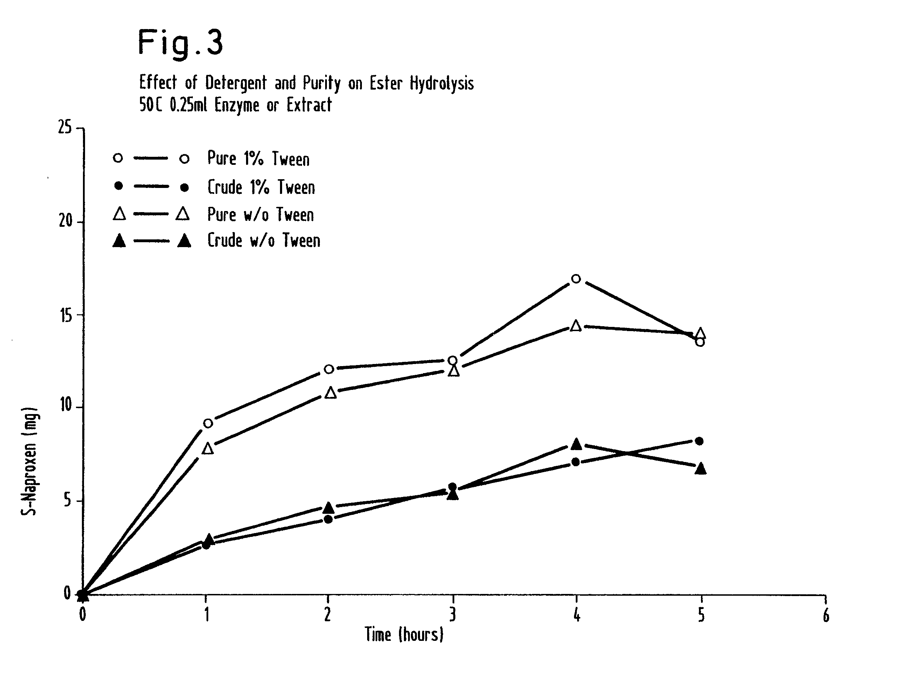

To determine if the P. fluorescens ester hydrolase of the invention required surfactants for full activity, R,S-naproxen ethyl ester hydrolysis was carried out at 50 °C with or without adding detergent or soy oil. The results indicated that the P. fluorescens ester hydrolase was equally active in the presence or absence of surfactant and there was no requirement for a surfactant such as soy oil, detergent or the like (Figure 3).

In a preferred practice of the invention, the purified cloned ester hydrolase was shown to hydrolyze to completion 30 gm/L of R-,S-naproxen ethyl ester in 5 hours at 50 °C, with or without the addition of a surfactant, such as the detergent Tween 80, while maintaining a product enantiomeric excess (ee) of greater than 97.5%. In contrast, as shown in Example 3, Table 1, none of the other commercial Pseudomonas lipases were active towards R-,S-naproxen ethyl ester under such conditions.

The cloned ester hydrolases of the invention can be readily purified from an E. coli cell lysate using various standard protein purification techniques, for example, affinity, ion exchange, size exclusion or hydrophobic chromatography. Active enzyme recovered from such purification techniques can be concentrated using ammonium sulphate, or alternatively, by lyophilization in the presence of sucrose.

An exemplary preparation and purification scheme comprises 1) growing the transformed E. coli cells in L broth and inducing with IPTG; 2) harvesting the culture by centrifugation; 3) resuspending the cell pellet in buffer followed by cell disruption; 4) centrifuging the cell lysate; and 5) purifying the enzyme by passage of the cell lysate over a two-step chromatographic column.

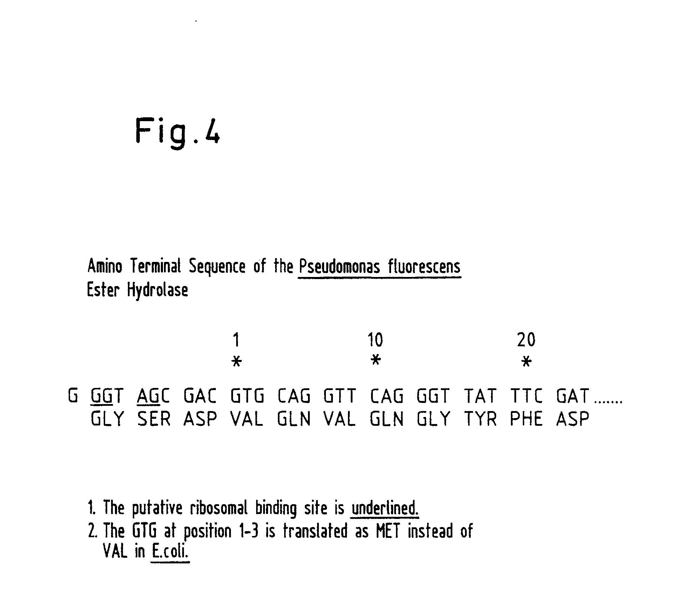

To further characterize the cloned ester hydrolase of the invention the DNA and amino acid sequence of the authentic ester hydrolase and the fused ester hydrolase were determined. Comparison between the protein and the DNA sequence (Figure 4) indicate that the translational start of the ester hydrolase gene is at least 450 base pairs (bp) downstream from the lac P-O. The authentic ester hydrolase does not appear to have a signal sequence. Also, a GTG (Val) is used instead of an ATG (Met) codon as the translational start site. A Shine-Delgarno sequence, i.e., ribosomal binding site, was identified 7 nucleotides upstream from the GTG start. The authentic ester hydrolase was found to have the following N-terminal sequence: Met-Gln-Val-Gln-Gly-Tyr-Phe-Asp-Leu-Arg-, which was determined to be encoded by the nucleotide sequence 5'GTGCAGGTTCAGGGTTATTTCGATCTTCGC3'. The complete sequence for the authentic ester hydrolase, 382 amino acids in length, was determined (Figure 10).

Replacement of the first five N-terminal amino acids of the ester hydrolase gene with the first eleven amino acids of lac Z, the beta galactosidase gene of the lac operon, resulted in a fusion protein, designated fused ester hydrolase, which retained full enzymatic activity. This result independently established the proper translational reading frame of the DNA sequence determined for the cloned ester hydrolase gene.

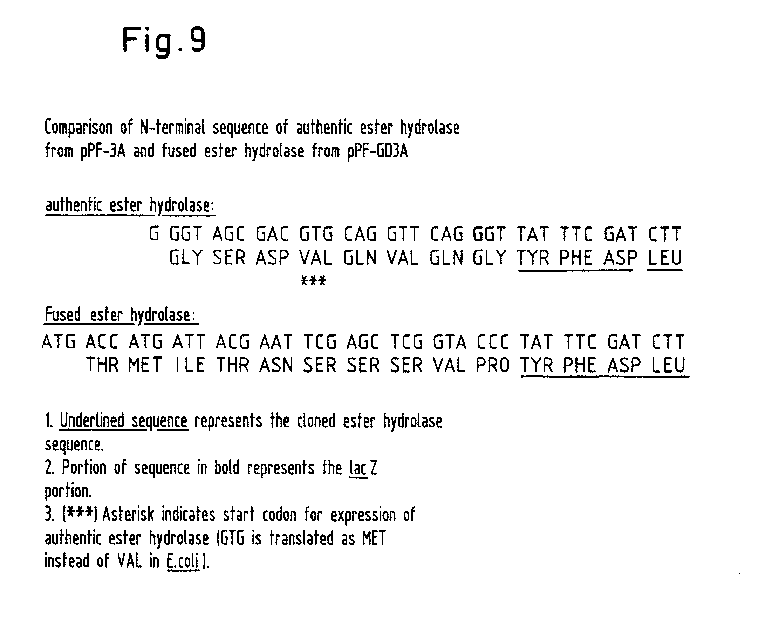

Additional studies on the cloned ester hydrolase resulting from the fusion of the lac Z with the gene for the ester hydrolase from pPF-GD3A indicated that an active ester hydrolase can be expressed from the fusion of the lac Z and the gene for authentic ester hydrolase. Thus it was shown that an active cloned ester hydrolase can be expressed when the lac Z is joined directly and in frame to the sixth P. fluorescens amino acid (Tyr), thereby eliminating the approximately 450 base pairs separating the promoter from the ester hydrolase gene (Figure 9). The complete sequence for this fused ester hydrolase, 386 amino acids in length, was determined (Figure 11). The N-terminal portion of the sequence comprises the nine amino acids encoding the lac Z fused in frame to the 1134 base pairs established as the minimum sequence required for the expression of an active fused ester hydrolase. The resulting 1161 base pairs (Figure 11) sets forth an apparent minimum sequence necessary to express an active fused ester hydrolase. (See Example 5 and 6 for more complete description of the sequencing experiments.)

In terms of enzyme stabilization, in addition to genetic modifications (protein engineering), one can chemically modify the enzyme as well. These include modification of surface amino-groups by alkylation or acylation (Torchillin, Biochim. Biophys. Acta. 567 :1,(1979)), intramolecular cross-linking (Torchillin, Biochim. Biophy. Acta , 522:277 (1977)), or enzyme immobilization which involves a multitude of different approaches (Chibata, J. Mol. Catal. 63 (Review Issue) (1986); Trevan: Immobilized Enzymes: Introduction and Application in Biotechnology , John Wiley, Chichester, UK, 1980)).

Employment of the P. fluorescens cloned ester hydrolase in the actual industrial production of naproxen can be carried out in many formats. For example, the enzyme can be added into a continuous stirred tank reactor. Likewise, it can be immobilized onto membranes. In either case, a suitable R-,S-naproxen ester substrate (typically a lower alkyl ester, e.g., the methyl, ethyl, isopropyl ester) will be introduced continuously into the reactor as a slurry.(Example 10) The actual residence time of the cloned ester hydrolase in the enzyme reactor will depend on both the substrate infusion rate as well as the removal rate of the final product. The enzymatic hydrolysis of the invention can be conducted in a continuous or batch mode. When using the authentic ester hydrolase, the reaction is generally carried out at the temperature range between 30 and 60°C, preferably between 45 and 55°C. Higher reaction temperatures may be used when the temperature-resistant ester hydrolase is employed as discussed earlier.

When using the authentic ester hydrolase in the reaction protocol, incubation temperature will most preferably be between 45-55°C with KOH added to maintain the proper pH (between 8.0 and 9.5) although many other bases are also suitable. S-naproxen salt, the product of the ester hydrolysis, is preferably removed from the process stream by passing through a series of filtration membranes that have different and specific molecular weight cut-offs. This avoids the entry of either the unreacted ester substrate or the cloned ester hydrolase into the final product. The final product can then be further purified by crystallization. The unreacted R-ester, as well as any residual S-ester enantiomer, can be recycled through a separate reactor in which both are racemized chemically. The resultant 50-50 racemic mixture of naproxen ester, as well as fresh R-,S-naproxen ester, can again be introduced into the bioreactors and the processing cycle repeated.

In order to improve the performance of the hydrolysis reaction for naproxen ester, it is desirable to develop P. fluorescens ester hydrolase mutants having improved performance characteristics in terms of rate of hydrolysis, chiral specificity and temperature stability. In particular, it is highly desirable to develop a thermally stable cloned ester hydrolase that can perform the hydrolysis reaction at 55°C or higher.

Mutagenesis experiments were carried out in an attempt to develop a more thermally stable P. fluorescens ester hydrolase mutant. A cloned ester hydrolase gene was transferred into bacteriophage M13mp19 (Messing, Gene 19 :269 (1982)) to simplify mutagenesis experiments and permit high throughput enzymatic assays. That is, thousands of phage plaques can be screened on an agar plate using an ester-hydrolase overlay assay (Higerd and Spizizen, J. Bacteriol. 114 :1184 (1973). M13 phage infected cells can be easily lysed with the addition of a few drops of chloroform. This enables one to make a large number of protein extracts without resorting to pelleting and sonicating of bacterial cells. When recombinant M13 phages containing the ester hydrolase gene were mutagenized with hydroxylamine, a number of temperature-resistant mutants were generated. In addition to chemical mutagenesis, other forms of mutagenesis include site-directed mutagenesis (Smith, Ann. Rev. Genet.19 :423 (1985)) to enhance thermal stability (Matthews, Biochemistry 26 :6885 (1987)), and the like, the methods of which are known to those skilled in the art.

Ester hydrolase recovered from the mutants, designated temperature-resistant ester hydrolases, formed as described above showed increased residual enzymatic activity after heat treatment at up to 65°C as discussed earlier.

## EXAMPLES

The examples which follow are illustrative and not limiting of the invention. Enzymes used in cloning experiments were obtained from commercial sources and were used substantially in accordance with the manufacturers instruction. Except where otherwise indicated, procedures such as DNA preparation, cleavage with restriction enzymes, ligation and transformation, were carried out essentially as described by Maniatis, et al ., supra .

Example 1. Molecular cloning of Pseudomonas ester hydrolase gene.

Pseudomonas fluorescens (ATCC#17550) DNA, partially digested with Sau3A, was ligated to Bam HI cleaved pUC19 (Yanisch-Perron, et al ., supra ) DNA. The ligated mixture was introduced into E. coli JM109 (Messing, supra ). Transformants were screened for ester hydrolase activity using a soft agar overlay assay (Higerd and Spizizen, supra ) and a number of the transformants were shown to be positive. Briefly, low melting agarose containing beta-naphthyl acetate and fast blue was spread over the bacterial colonies. Development of blue color occurs when β-naphthol released by the ester hydrolase positive clones react with fast blue.

The positive clones were then grown overnight in L broth (10g/L Tryptone, 5 g/L Yeast extract and 10

g/L NaCl) supplemented with either the R- or the S- isomer of naproxen methyl ester (1 mg/ml in soy oil). The culture broths (1 ml each) were extracted with 1 ml saturated ammonium sulphate, 50 µl concentrated phosphoric acid and 1.5 ml concentrated ethyl acetate (J.T. Baker, Phillipsburg. NJ). After centrifugation for phase separation, the organic phase was analyzed on thin layer chromatography plates (TLC) plates. The TLC plates were developed by running three times in 100% hexane to remove soy oil and once in 95% $CH_2Cl_2$, 5% methanol and 0.05% ammonium hydroxide to resolve naproxen from its ester. Multiple clones showed strong preference for the S-naproxen ester. One of the positive clones designated pPF-3A, was subjected to further analysis.

Example 2. Characterization of the P. fluorescens ester hydrolase gene.

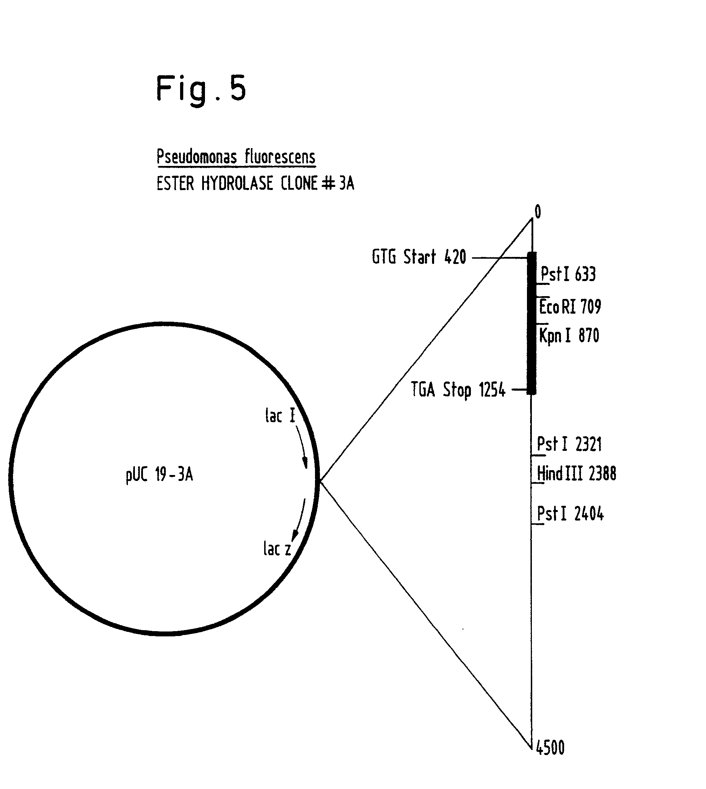

Plasmid pPF-3A (Figure 5) has a DNA insert of 4.5 kb. A sample of E. coli carrying this plasmid has been deposited with the American Tissue Culture Collection (ATCC) and has been assigned the designation ATCC #68083.

Subcloning experiments indicated the authentic ester hydrolase gene is encoded within a 2.4 kb Hind III fragment. Plasmid pPF-3A DNA was cleaved with Hind III and ligated to Hind III cleaved pUC18 or pUC 19 vector DNAs. Positive ester hydrolase clones were identified as described in Example 1. Plasmid DNAs were prepared from the positive colonies and characterized in detail using several restriction enzymes. Expression of the hydrolase activity in pUC18 and pUC19 appeared to be entirely orientation dependent, indicating that the endogenous Pseudomonas promoter, if present, is not functional in E. coli . One of the ester hydrolase positive clones, cloned in plasmid pUC18, was designated pPF-18-1. Further deletion studies accurately established the position of the ester hydrolase gene within the 2.4 kb Hind III fragment.

Example 3. Characterization of the ester hydrolase from pPF-3A

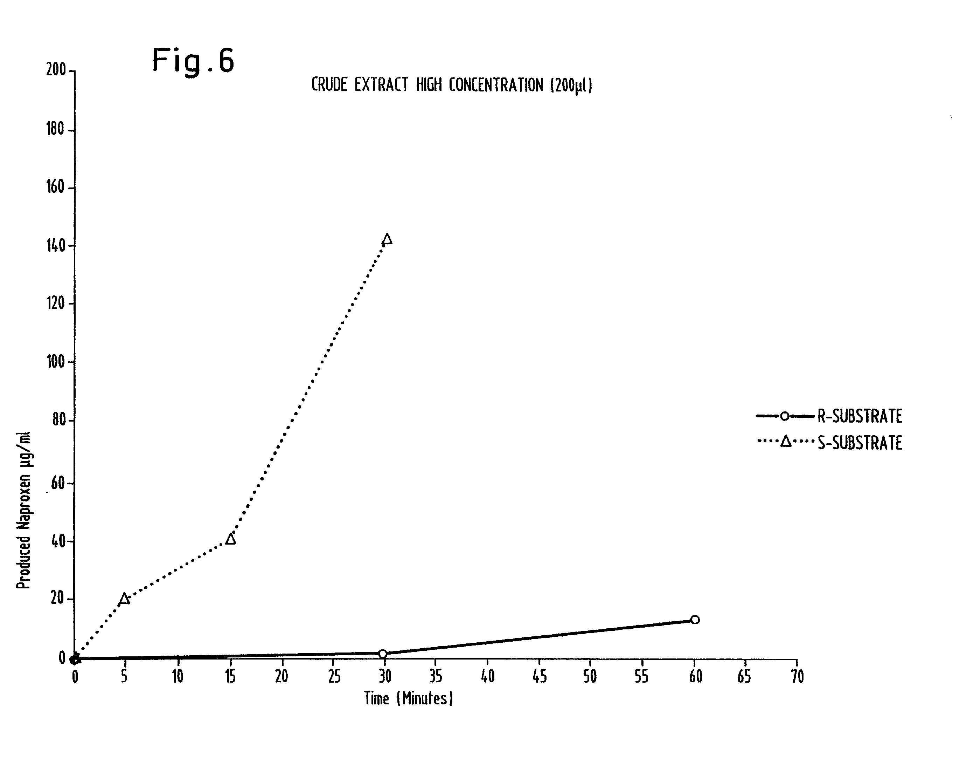

A crude protein extract was prepared from E. coli cells carrying pPF-3A and assayed for chiral specific esterolytic activity. Essentially, an overnight bacterial culture of pPF-3A (1.5 L) was pelleted and resuspended in 100 ml of Tris HCl, pH 8.0. After sonication, the extract was cleared by low speed centrifugation (12,000 x g) and the supernatant recovered. R- and S- methyl esters of naproxen (20 mg/ml) were individually presented to the crude protein extract. Figure 6 shows the hydrolysis results which evidenced the strong preference of pPF-3A for the S-naproxen-ester.

The level of authentic ester hydrolase produced by E. coli cells carrying pPF-3A could be modulated by the addition of isopropylthio-galactoside (IPTG), an inducer for the E. coli operon. At least a 3 fold increase in esterolytic activity was observed when the bacterial culture was induced with IPTG (1 mM).

To prepare purified samples of the P. fluorescens ester hydrolase, E. coli cells harboring pPF-3A were grown in L broth and induced with 1 mM IPTG. 5 hours post-induction, the culture (10 liters) was harvested by centrifugation. The pellet was dissolved in 2 L of buffer and disrupted using a Gaulin Disruptor. The cell lysate was then centrifuged and the supernatant was loaded onto a DEAE-Sephacel column previously equilibrated with 50 mM Tris-HCl, pH 9. The column was eluted with a linear salt gradient (0-0.5 M NaCl in 50 mM Tris-HCl, pH9). Active fractions (0.3-0.4 M NaCl) were pooled and precipitated with 70% ammonium sulphate saturation. After centrifugation, the pellet was dissolved in 150 ml of 50 mM Tris-HCl, pH 9, and applied onto a G-100 gel filtration column. Active authentic ester hydrolase, which eluted with an apparent molecular weight of 30 kD was recovered and concentrated (either by ammonium sulphate precipitation or lyophilization). SDS-polyacrylamide gel electrophoresis of the purified enzyme sample showed a major band at approximately 33 kD.

Activity staining of the cloned ester hydrolase and other commercial P. fluorescens lipases (Amano: Lipase P "Amano"-PR-13 derived from P. fluorescens and Lipase AK "Amano"-PR-14 derived from Pseudomonas sp . ;, Fluka: Lipoprotein Lipase from Pseudomonas sp.(EC 3.1.1.34) (9004-02-8) and from P. fluorescens (EC 3.1.1.3) (9001-62-1); Boehringer Mannheim: Lipase from Pseudomonas species (Triacylglycerol acylhydrolase, EC 3.1.1.3) and Sigma: Type XIII from Pseudomonas species (L9518)), which were resolved on a non-denaturing polyacrylamide gel, indicated that the cloned ester hydrolase was distinctly different from the other commercial Pseudomonas enzymes in terms of its differential migration in a non-denaturing gel system (Figure 1).

Thermal stability of the authentic ester hydrolase was determined by incubating the enzyme at 5 degrees intervals between 40° C and 55° C. and subsequently assaying for residual activity. Figure 2 shows the half life of the enzyme to be greater than 4 hours at 50° C.

The cloned ester hydrolase, either in its crude or purified form, requires no surfactant for full activity (Figure 3). At 30 gm/L of naproxen ethyl ester, the purified cloned ester hydrolase was able to hydrolyze to completion, all available substrate in 5 hours at 50° C, with or without the addition of the detergent Tween, while maintaining the high ee of greater than 97.5%. As shown in Table 1, none of the other commercial Pseudomonas lipases were active towards naproxen ethyl ester under these conditions.

Table 1

| | |
|---|---|
| Substrate level: R-,S-Naproxen ethyl ester (10 mg/ml)<br>Reaction volume: 0.5 ml<br>Reaction temperature: 50° C.<br>Reaction time: 1 hour<br>Product analysis: HPLC | |
| Enzyme | % Hydrolysis |
| Fluka (EC 3.1.1.34) ( P. species ) | 0.035 |
| Fluka (EC 3.1.1.3) ( P. fluorescens ) | 0.05 |
| Sigma ( P. species ) | 0.09 |
| Boehringer Mannheim ( C cylindracea ) | 0.05 |
| Amano Lipase P ( P. fluorescens ) | 0.04 |
| Amano AK ( P. species ) | 0.06 |
| Cloned ester hydrolase | 6.40 |
| Units of enzyme were equivalent based on vendors' specification | |

Example 4 N-terminal amino acid sequence determination.

The N-terminal amino acid sequence of the cloned ester hydrolase from pPF-3A was determined using a micro-sequencing technique. In particular, a purified preparation of the enzyme prepared according to Example 3, was electrophoresed on a SDS-polyacrylamide gel. The resolved protein band(s) were then electro-blotted onto an Immobulon filter (Millipore Corporation, Medford, Ma. USA) and the protein band of interest was cut out and subjected to standard micro-sequencing technique (Matsudira, J. Biol. Chem. 262 : 10035 (1987)). The products were then analyzed on an automated gas-phase microsequenator (Applied Biosystem Inc., Foster City, Ca. USA) using the methods as described by Hunkapellier et al ., Meth. Enz. 91 :399 (1983) . The cloned P. fluorescens ester hydrolase was found to have the following N-terminal sequence: Met-Gln-Val-Gln-Gly-Tyr-Phe-Asp-Leu-Arg- (Figure 4).

Concurrently, the DNA sequence of the 2.4 kb Hind III insert of pPF-18-1 was also determined using the dideoxy-sequencing procedure as described by Sanger, Proc. Natl. Acad. Sci. U.S.A. 74 :5463 (1977)). Translation of the DNA sequence in all three reading frames identified a stretch of nucleotides: 5′GTGCAGGTTCAGGGTTATTTCGATCTTCGC3′, which encode the aforementioned ten N-terminal amino acids (see Figure 4). Of note is that the ester hydrolase gene does not appear to have a signal sequence, and it utilizes a GTG codon (normally for Val) instead of ATG (Met) for translational initiation. A Shine-Delgarno like sequence (ribosomal binding site) was identified 7 nucleotides upstream from the GTG start.

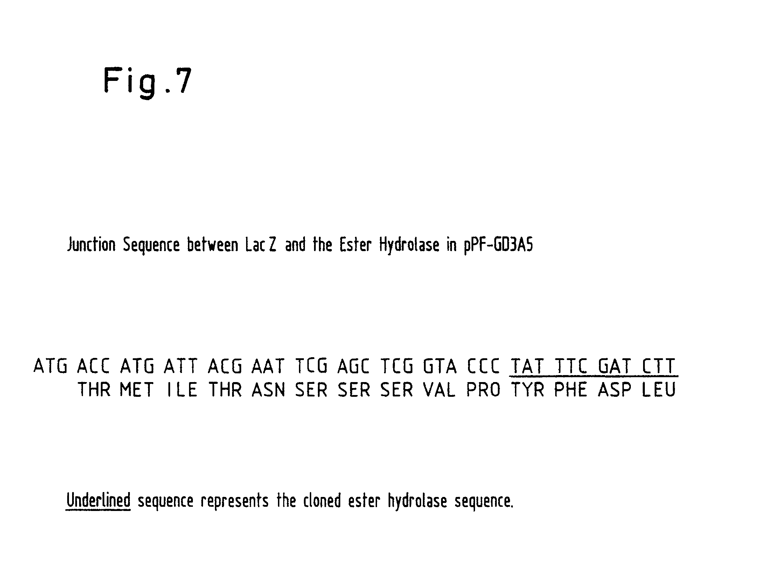

Initial experiments identified a single Mbo II site within the authentic ester hydrolase gene, 5 amino acids from the amino terminus. Cleavage of the 2.4 kb Hind III DNA fragment with Mbo II released a DNA fragment of approximately 1100 base pairs. The Mbo II fragment was cloned into pUC18 previously cleaved with SmaI. A positive ester hydrolase clone, designated pPF-GD3A5, was identified and its plasmid DNA was prepared and sequenced. Figure 7 shows the nucleotide and amino acid sequence around the lac Z-ester hydrolase fusion junction. Studies using this fused ester hydrolase evidenced that the first few amino acids of the cloned ester hydrolase were dispensable and that the previously ascribed translational reading frame of the cloned ester hydrolase gene was indeed correct.

Example 5 Studies to determine the minimum sequence necessary to express an active fused ester hydrolase

The extent of the 5′-carboxyl coding region was determined by unidirectional truncation. Exonuclease III was used to digest the coding region of the carboxyl portion of the cloned ester hydrolase gene beginning at the downstream proximal Sal I site (Maniatis, *supra* ).

Briefly, 50 µg of the esterase DNA from pPF-GD3A was digested with 250 Units of Sal I enzyme and then 200 Units of Sph I (New England Biolabs, Beverly, MA.). 10 µg Sal I SphI treated DNA was treated with 14 Units of Exonuclease III in 60 µl digestion buffer at 37° C. Aliquots of 800 ng were removed at one minute intervals, chilled on ice, phenol-$CHCl_3$ extracted and ethanol precipitated. Each 800 ng aliquot was resuspended in Mung Bean nuclease buffer and Biolabs) in 10 µl volume at 30° C for 30 minutes. Concentration of enzyme and buffers used for dilution were carried out according to anufacturer's recommended procedure. Samples were extracted with phenol-$CHCl_3$ and ethanol precipitated (Maniatis, *supra* ).

400 ng of each 1, 2 and 3′ Exonuclease III timepoint samples were pooled. 200 ng of the now blunt-ended insert was ligated to 500 ng Xba I amber stop termination linker (Pharmacia, Piscataway, NJ) using 2 Weiss Units T4 DNA ligase (Boehringer Mannheim, West Germany) in 21 µl blunt end ligase buffer (Maniatis, *supra* ) at 14° C overnight.

Following the overnight incubation, the ligation mix was digested with a total of 60 Units of Xba I for 4.5 hours at 37° C. The treated sample was purified over a Sephracryl S-400 column (Promega, Madison, WI). The sample was then recircularized in 30 µl cohesive end ligation buffer (Maniatis, *supra* ) using 0.1 weiss Unit T4 DNA ligase at 14 °C for 4 hours. 15 µl of recircularized DNA was used to transform 100 µl *E. coli* Competent JM109 cells (Stratagene, Lajolla, CA) and plated on Xgal IPTG Amp LB plates.

Analysis of the fused ester hydrolase negative clones showed that removal of as few as 24 nucleotides 5′ to the TGA stop codon at bp 1161 (see Figure 11) inactivated expression of the cloned gene.

Example 6 Determination of the complete sequence for the cloned ester hydrolase

The DNA sequence for the cloned ester hydrolase (the fused ester hydrolase and the authentic ester hydrolase) was obtained using an Applied Biosystem 373A automated DNA sequencing machine (Applied Biosystems Inc., Foster City, CA) using two alternative procedures recommended by the vendor. The sequencer separates, detects and identifies fluorescently labeled DNA molecules from dideoxy sequencing reactions.

The first sequencing procedure employed the Tag polymerase protocol provided in the Applied Biosystem User Manual. This procedure utilized a dye labeled primer and was used mainly to determine DNA sequences most proximal to the cloning vector (plasmid pUC18).

The second procedure employed a double-stranded cycling procedure essentially as described by the vendor except 1.5 µl of dNTP mix was used instead of 1 µl. Synthetic oligonucleotides, the sequence of which were based on the results from the Tag polymerase sequencing procedure, were used as primers for the double-stranded cycling procedure. The Polymerase Chain Reaction (PCR) reactions were performed using a Perkin Elmer-Cetus DNA Thermal Cycler (Norwalk, CT) using conditions specified by the manufacturer, i.e., 30 step cycles of 98° C for second, 60° C for 2 minutes.

Purification of extension products was performed according to Applied Biosystem, Inc.'s protocol using a G-50 Sephadex spin column (BioRad, Hercules, CA). This was followed by ethanol precipitation. The PCR products were loaded onto the Applied Biosystems 373A sequencing machine.

Using this procedure the complete sequence for the authentic ester hydrolase (Figure 10) and the fused ester hydrolase (Figure 11) were determined.

Example 7 Studies on the cloned ester hydrolase to verify the unique characteristics of the cloned enzyme

Most of the known commercially available lipases characterized thus far contain the consensus amino acid sequence Gly-X-Ser-X-Gly at their substrate binding regions (Boel, *Lipids* 23 :701 (1988). This consensus amino acid sequence is noticably absent in the *P. fluorescens* cloned ester hydrolase. To determine if the cloned ester hydrolase is an efficient lipase, the enzyme was assayed for lipase activity using a lipase assay kit (Sigma Chemical, St. Louis, MO). This assay is based on the methods of Fiereck ( *Clin. Chem. Acta.* 13 :352 (1966) and uses olive oil as the assay substrate. The results are shown in Table

2.

Table 2

| Enzyme | Units of Hydrolysis |
|---|---|
| Fluka (EC 3.1.1.3) ( P. fluorescens ) | 15 |
| Sigma ( P. species ) | 38 |
| Boehringer Mannheim ( C cylindracea ) | >85 |
| Amano Lipase P ( P. fluorescens ) | 18 |
| Amano AK ( P. species ) | 18 |
| Cloned ester hydrolase | 0 |
| Units of enzyme were equivalent based on vendors' specifications. Units of hydrolysis are defined as Sigma-Tietz Units according to Vendor's protocol (Sigma Chemical, St. Louis, MO). | |

These results corroborate those set forth in Example 3 verifying that the cloned ester hydrolase is distinct from any known commercially available enzymes.

Example 8 Cloning of ester hydrolase genes from Pseudomonas mendocina and stutzeri.

Ester hydrolase positive clones from P. mendocina (SCC#3-180) and P. stutzeri (ATCC#17588) were constructed and identified as described in Example 1. The ester hydrolases produced by selected ester hydrolase clones were found to hydrolyze naproxen ester in a chiral specific manner. Using Southern blot hybridization (Southern, J. Mol. Biol. 98 ;503 (1975)) the ester hydrolase genes from these two species were shown to be highly homologous to the authentic ester hydrolase gene isolated from P. fluorescens .

Example 9 Mutagenesis of the ester hydrolase gene.

The 2.4 kb Hind III fragment of pPF-3A was cloned into M13mp19 (see Maniatis et al., supra ). Ester hydrolase positive phage plaques were identified using the soft agar overlay assay described in Example 1. A high titer phage stock was prepared from one of the recombinant phages, designated M13-PF10-2.

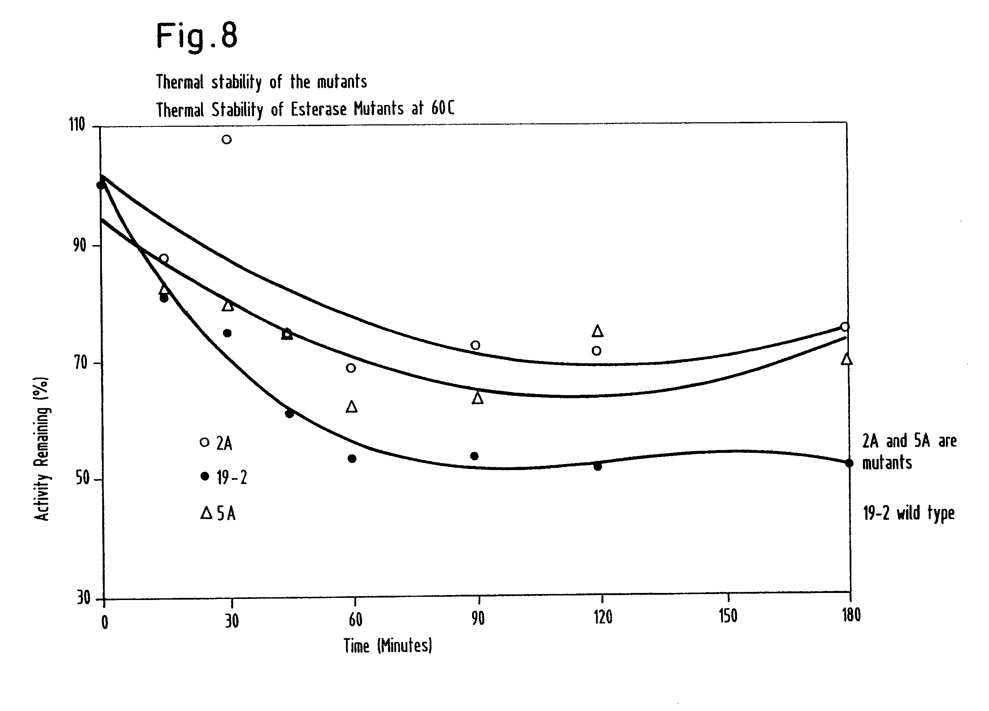

$3 \times 10^{12}$ pfu's of M13-PF10-2 were mutagenized with hydroxylamine (0.25 M) for eight hours. The mutagenized phages were then diluted and plated onto an E. coli lawn embedded in soft agar. After overnight growth at 37° C, the agar plates were wrapped in foil and placed in 37°, 55° and 60° C ovens. After three hours incubation, the plates were retrieved and assayed for residual esterase activity. This was done by overlaying the phage plaques with agarose containing fast blue and beta-naphthyl acetate as described in Example 1. Phage plaques which retained considerable enzymatic activity were picked and propagated. Two of these, designated M13-PF2A and M13-PF5A were further characterized. Hind III inserts of M13-PF2A and 5A were re-introduced into plasmid pUC18. Progeny clones, designated pPF-2A and pPF-5A were likewise shown to retain good ester hydrolase activity after incubation at 55° C and 60° C for three hours. Protein extracts were prepared and the thermal stability of the temperature-resistant ester hydrolase was evaluated as described in Example 2. Figure 8 shows the half lives of pPF-2A and pPF-5A were substantially extended at 60° C versus their wild type parent pPF-18-1.

Example 10 Use of the Pseudomonas cloned ester hydrolase in a bioreactor for production of enantiospecific S-naproxen

To a slurry of finely ground ethyl or methyl ester of R,S-naproxen in water (50-250 gm per liter) was added the cloned ester hydrolase 50-250 units/gm ester). Optionally, a non-ionic surfactant may be added at a concentration of 0.1 to 2.0% to assist in the dispersion of the ester. The reaction mixture was maintained at a temperature in the range of 35-45° C. pH was maintained in the range of 6-10 by the addition of base. Reaction completion was determined by monitoring the uptake of base or by chromatographic analysis of the reaction mixture. When the reaction was deemed complete (4-48 hrs) by the uptake of base, unreacted ester was removed by filtration and the filtrate, containing naproxen as its salt, was treated with acid and filtered. The collected S-naproxen was analyzed for purity and chiral specificity using mass spectrometry, high performance liquid chromatography (HPLC) and other such recognized methods of analysis. Further purification of the filtered naproxen may be carried out as required.

The above description and examples serve to fully disclose the invention including preferred embodiments thereof. Modifications obvious to those of ordinary skill in molecular biology, protein chemistry, biochemical engineering and related sciences are intended to be within the scope of the following claims.

## Claims

1. A DNA molecule comprising a gene for ester hydrolase obtained from Pseudomonas fluorescens and encoded within a 2.4 kb Hind III fragment of the DNA molecule.

2. The DNA molecule of claim 1 comprising a gene encoding an authentic ester hydrolase having an N-terminal amino acid sequence as follows:

$_1$Met-Gln-Val-Gln-Gly-$_6$Tyr-Phe-Asp-Leu-Arg-...

wherein the start codon for expression of the authentic ester hydrolase is GTG but is translated as a methionine (Met) rather than a valine (Val).

3. The DNA molecule of claim 2 cloned and expressed in E. coli .

4. The DNA molecule of claim 2 which encodes an authentic ester hydrolase having the nucleotide sequence and corresponding amino acid sequence as set forth in Figure 10.

5. The DNA molecule of claim 1 encoding an authentic ester hydrolase having high thermal stability and enantiospecificity for S-naproxen esters.

6. The DNA molecule of claim 5 which encodes an authentic ester hydrolase capable of enantiospecifically catalyzing the hydrolysis of R-,S-naproxen esters to produce S-naproxen.

7. The DNA molecule of claim 6 in which the S-naproxen is formed in greater than 97% enantiomeric excess.

8. The DNA molecule of claim 5 which encodes an authentic ester hydrolase that is stable and enzymatically active at temperatures from 45° C to 60° C.

9. The DNA molecule of claim 8 which encodes an authentic ester hydrolase that is stable and enzymatically active at 55° C.

10. A gene which encodes the amino acid sequence for authentic ester hydrolase as set forth in Figure 10.

11. The gene of claim 10 which encodes the amino acid sequence for a derivative of authentic ester hydrolase wherein the amino acid sequence encoded by the gene has at least a 75% homology to amino acid sequence for authentic ester hydrolase as set forth in Figure 10.

12. The DNA molecule of claim 1 wherein cloning into E. coli is carried out under the control of a heterologous bacterial promoter.

13. The DNA molecule of claim 12 wherein the cloning into E. coli is carried out under the control of the lac promoter inserted upstream from the DNA sequence of the cloned ester hydrolase gene.

14. The DNA molecule of claim 12 comprising a gene encoding a fused ester hydrolase having an N-terminal amino acid sequence as follows:

Met-Ile-Thr-Asn-Ser-Ser-Ser-Val-Pro-Tyr-Phe-Asp-Leu-Arg

resulting from the fusion of the lac Z portion of the lac promoter with the gene encoding authentic ester hydrolase wherein lac Z is fused in frame to the sixth amino acid of the authentic ester hydrolase gene.

15. The DNA molecule of claim 12 which encodes a fused ester hydrolase having the nucleotide sequence and corresponding amino acid sequence as set forth in Figure 11.

16. The DNA molecule of claim 1 which encodes a fused ester hydrolase having high thermal stability and enantiospecificity for S-naproxen esters.

17. The DNA molecule of claim 16 which encodes a fused ester hydrolase capable of enantiospecifically catalyzing the hydrolysis of R-,S-naproxen esters to produce S-naproxen.

18. The DNA molecule of claim 17 in which the S-naproxen is formed in greater than 97% enantiomeric excess.

19. The DNA molecule of claim 16 which encodes a fused ester hydrolase that is stable and enzymatically active at temperatures from 45° C to 60° C.

20. The DNA molecule of claim 19 which encodes an fused ester hydrolase that is stable and enzymatically active at 55° C.

21. A gene which encodes the amino acid sequence for fused ester hydrolase as set forth in Figure 11.

22. The gene of claim 21 which encodes the amino acid sequence for a derivative of fused ester hydrolase wherein the amino acid sequence encoded by the gene has at least a 75% homology to the amino acid sequence for fused ester hydrolase as set forth in Figure 11.

23. The DNA molecule of claim 1 comprising a gene encoding a temperature-resistant ester hydrolase wherein the sequence for authentic ester hydrolase has been mutated to confer temperature-resistance to the expressed enzyme.

24. The DNA molecule of claim 23 cloned and expressed in E. coli .

25. The DNA molecule of claim 23 encoding an temperature-resistant ester hydrolase having high thermal stability and enantiospecificity for S-naproxen esters.

26. The DNA molecule of claim 25 which encodes a temperature-resistant ester hydrolase capable of enantiospecifically catalyzing the hydrolysis of R-,S-naproxen esters to produce S-naproxen.

27. The DNA molecule of claim 26 in which the S-naproxen is formed in greater than 97% enantiomeric excess.

28. The DNA molecule of claim 26 which encodes a temperature-resistant ester hydrolase that is stable and enzymatically active at temperatures from 45° C to 65° C.

29. The DNA molecule of claim 28 which encodes a temperature-resistant ester hydrolase that is stable and enzymatically active at 62° C.

30. The DNA molecule of claim 1 encoding a cloned ester hydrolase having high thermal stability and enantiospecificity for S-naproxen esters.

31. The DNA molecule of claim 30 wherein the cloned ester hydrolase is selected from the group authentic ester hydrolase, fused ester hydrolase or temperature-resistant ester hydrolase.

32. The DNA molecule of claim 30 which encodes a cloned ester hydrolase capable of enantiospecifically catalyzing the hydrolysis of R-,S-naproxen esters to produce S-naproxen.

33. The DNA molecule of claim 32 in which the S-naproxen is formed in greater than 97% enantiomeric excess.

34. The DNA molecule of claim 30 which encodes a cloned ester hydrolase that is stable and active at temperatures from 45° C to 65° C.

35. A DNA molecule comprising a gene for ester hydrolase obtained from a microbial host selected from the group Pseudomonas fluorescens, Pseudomonas mendocina and Pseudomonas stutzeri .

36. The DNA molecule of claim 35 which encodes a cloned ester hydrolase that is stable and active at temperatures from 45° C to 65° C.

37. A DNA molecule comprising a gene for a cloned ester hydrolase obtained from the microbial host Pseudomonas fluorescens and encoded within a 2.4 kb Hind III fragment of the DNA molecule or temperature resistant mutants thereof downstream to regulatory elements functional in E. coli .

38. The DNA molecule of claim 37 wherein cloning into E. coli is carried out under the control of a heterologous bacterial promoter.

39. The DNA molecule of claim 38 wherein the cloning into E. coli is carried out under the control of the lac promoter inserted upstream from the DNA sequence of the cloned ester hydrolase gene.

40. A process for the production of S-naproxen wherein a cloned ester hydrolase is used to enantioselectively catalyze the hydrolysis of R,S-naproxen alkyl ester, which process comprises:

(1) producing in E. coli a cloned ester hydrolase from Pseudomonas fluorescens having improved performance characteristics

(2) presenting the cloned ester hydrolase either as free soluble enzyme or in its immobilized form to the R-,S-naproxen ester;

(3) hydrolyzing the R-,S-naproxen ester; and

(4) collecting S-naproxen.

41. The process of claim 40 wherein the cloned ester hydrolase is selected from the group authentic ester hydrolase, fused ester hydrolase or temperature-resistant ester hydrolase.

42. The process of claim 40 wherein the cloned ester hydrolase controls the rate and enantiospecificity of the hydrolysis reaction.

43. The process of claim 42 wherein the cloned ester hydrolase catalyzes the hydrolysis reaction at temperature range from 30° C to 65° C.

44. The process of claim 42 wherein the hydrolysis of naproxen alkyl ester using the cloned ester hydrolase yields S-naproxen in an enantiomeric excess of greater than 97%.

45. An ester hydrolase having the amino acid sequence as set forth in Figure 10, or a sequence related thereto by substitution, addition or deletion of one or more amino acids and is capable of effecting the hydrolysis of R,S naproxen ester to S-naproxen in an enantiomeric excess (ee) of greater than 97%.

46. An ester hydrolase having the amino acid sequence as set forth in Figure 11, or a sequence related thereto by substitution, addition of deletion of one or more amino acids and is capable of effecting the hydrolysis of R,S naproxen ester to S-naproxen in an enantiomeric excess (ee) of greater than 97%.

47. The ester hydrolase of claim 45 or 46 wherein the ester hydrolase catalyzes the hydrolysis reaction at a temperature range from 30°C to 65°C.

48. A process for the production of S-naproxen or a pharmaceutically acceptable salt thereof comprising subjecting an R,S-naproxen ester to the action of the ester hydrolase of claim 45 or 46 to afford S-naproxen.

49. A process for producing S-naproxen from R, S-naproxen alkyl ester, which process comprises:

reacting the R,S-naproxen alkyl ester with an ester hydrolase of claim 45 or 46 for a time sufficient to form S-naproxen in an enantiomeric excess of greater than 97%, and

collecting the resulting naproxen.

## Sequence Listing SEQ ID NO: 1
SEQUENCE TYPE: Nucleotide with corresponding protein
SEQUENCE LENGTH: 1149 base pairs STRANDEDNESS: single
TOPOLOGY: linear
MOLECULE TYPE: genomic DNA ORIGINAL SOURCE
ORGANISM: Pseudomonas fluorescens
IMMEDIATE EXPERIMENTAL SOURCE
NAME OF CELL LINE: Escherichia coli PROPERTIES: ester hydrolase gene

```
Met
GTG CAG GTT CAG GGT TAT TTC GAT CTT CGC TTC GAA GCG GTT    42
Val Gln Val Gln Gly Tyr Phe Asp Leu Arg Phe Glu Ala Val
                5                   10
CGC GAT GCG TTT GCC GCA TTG TTC GAC GGC ACC CAG CAA CGC    84
Arg Asp Ala Phe Ala Ala Leu Phe Asp Gly Thr Gln Gln Arg
15                  20                  25
GGC GCC GCA CTC TGT GTG CAG ATC GGC GGC GAA ACG GTG GTC   126
Gly Ala Ala Leu Cys Val Gln Ile Gly Gly Glu Thr Val Val
    30                  35                  40
GAT CTG TGG GCT GGC GTC GCC GAC AAC CAG GGC GAG CAG GCC   168
Asp Leu Trp Ala Gly Val Ala Asp Asn Gln Gly Glu Gln Ala
        45                  50                  55
TGG CAC AGC GAC ACG CTG GTC AAC CTG TTT TTC TGC ACC AAG   210
Trp His Ser Asp Thr Leu Val Asn Leu Phe Ser Cys Thr Lys
            60                  65                  70
ACC TTT ACC GCG GTG GCC GCG CTG CAG CTG GTC GAG GAG GGC   252
Thr Phe Thr Ala Val Ala Ala Leu Gln Leu Val Glu Glu Gly
                75                  80
AAG CTG GAA CTG GAT GCC CCG GTC GCG CGG GTC TGG CCG GAA   294
Lys Leu Glu Leu Asp Ala Pro Val Ala Arg Val Trp Pro Glu
85                  90                  95
```

```
TTC GCG GCC AAT GGC AAG GAA TCG ATA ACC TTG CGC CAG CTG   336
 Phe Ala Ala Asn Gly Lys Glu Ser Ile Thr Leu Arg Gln Leu
     100                 105                 110
 CTT TGT CAT CGC GCC GGG CTG CCG GCC ATT CGC CGG CCG CTG   378
 Leu Cys His Arg Ala Gly Leu Pro Ala Ile Arg Arg Pro Leu
         115                 120                 125
 GCG CCC GAG GCA CTC TAC GAC TGG ACG TGC ATG ACC GAC GCG   420
 Ala Pro Glu Ala Leu Tyr Asp Trp Thr Cys Met Thr Asp Ala
             130                 135                 140
 CTG GCC GCC GAG GAG CCC TGG TGG GCA CCG GGT ACC GAC CAG   462
 Leu Ala Ala Glu Glu Pro Trp Trp Ala Pro Gly Thr Asp Gln
                 145                 150
 GGT TAC GCC GCC ATG ACC TAC GGC TGG CTG GTC GGG GAG CTG   504
 Gly Tyr Ala Ala Met Thr Tyr Gly Trp Leu Val Gly Glu Leu
 155                 160                 165
 CTG CGT CGT GTC GAT GGC TGC GGA GCG GGC GAG TCG ATC GTT   546
 Leu Arg Arg Val Asp Gly Cys Gly Ala Gly Glu Ser Ile Val
     170                 175                 180
 CGT CGC ACC GCG GCG CCC CTG GGG CTG GAC TTC CAT GTT GGC   588
 Arg Arg Thr Ala Ala Pro Leu Gly Leu Asp Phe His Val Gly
         185                 190                 195
 CTG GAC GAC AGC CAG GCC GAT CGC GTC GCT TAC CTG ACC AGG   630
 Leu Asp Asp Ser Gln Ala Asp Arg Val Ala Tyr Leu Thr Arg
             200                 205                 210
 ACC AAG AAC GAC TTC GGC GAC GCT TGC GCC CAG CGT CTG TTG   672
 Thr Lys Asn Asp Phe Gly Asp Ala Cys Ala Gln Arg Leu Leu
                 215                 220
 AAG GCG CTG ATG AGT GAT CCT GCA TCC ATC AGC GCC CGC GCT   714
 Lys Ala Leu Met Ser Asp Pro Ala Ser Ile Ser Ala Arg Ala
 225                 230                 235
 TTC AAC AAT CCG CCA TCC ATC ATG AGC AGC GGC AAC AAG CCG   756
 Phe Asn Asn Pro Pro Ser Ile Met Ser Ser Gly Asn Lys Pro
     240                 245                 250
 GAA TGG CGA CGC ATG GCG CAG CCG GCG GCC AAC GGC CAC GGC   798
 Glu Trp Arg Arg Met Ala Gln Pro Ala Ala Asn Gly His Gly
         255                 260                 265
```

```
AAC GCG CGC TCG CTG GCC GGC TTG TAT ACC GGT TTG CTT CAG    840
Asn Ala Arg Ser Leu Ala Gly Leu Tyr Thr Gly Leu Leu Gln
            270                 275                 280
GGC AGG CTG CTC GAT GAG GCG GTC CTG CGT GAG ATG ACC CAT    882
Gly Arg Leu Leu Asp Glu Ala Val Leu Arg Glu Met Thr His
                285                 290
GAG CAC AGC GCT GGC GAG GAC CGT ACC CTG TTG GCC TCG ACG    924
Glu His Ser Ala Gly Glu Asp Arg Thr Leu Leu Ala Ser Thr
295                 300                 305
CGC TTC GGC CTG GGT TGC TGG CTC GAT CAG CCT GAC GTT GCC    966
Arg Phe Gly Leu Gly Cys Trp Leu Asp Gln Pro Asp Val Ala
    310                 315                 320
AAT GCG ACG TTC GCC ATG GGG CCG CGC GCG TTC GGC CAT CCA   1008
Asn Ala Thr Phe Ala Met Gly Pro Arg Ala Phe Gly His Pro
        325                 330                 335
GGT GCG GGT GGC TGC ATT GGC TTC GCC GAT CCG GAG CGT GAA   1050
Gly Ala Gly Gly Cys Ile Gly Phe Ala Asp Pro Glu Arg Glu
            340                 345                 350
CTG GGG TTC GGC TTC GTC ACC AAT ACT CTC GGG CCC TAT GTG   1092
Leu Gly Phe Gly Phe Val Thr Asn Thr Leu Gly Pro Tyr Val
                355                 360
CTG ATG GAC CCG AGA GCG CAA TCG CTG GCT CGC TGC GTA GCC   1134
Leu Met Asp Pro Arg Ala Gln Ser Leu Ala Arg Cys Val Ala
365                 370                 375
GCC TGC CTG CAT TGA                                       1149
Ala Cys Leu His  stop codon
    380
```

SEQ ID NO: 2
SEQUENCE TYPE: Nucleotide with corresponding protein
SEQUENCE LENGTH: 1161 base pairs STRANDEDNESS: single
TOPOLOGY: linear
MOLECULE TYPE: genomic DNA ORIGINAL SOURCE
ORGANISM: Pseudomonas fluorescens
IMMEDIATE EXPERIMENTAL SOURCE
NAME OF CELL LINE: Escherichia coli PROPERTIES: fused ester hydrolase gene

```
ATG ATT ACG AAT TCG AGC TCG GTA CCC TAT TTC GAT CTT CGC    42
Met Ile Thr Asn Ser Ser Ser Val Pro Tyr Phe Asp Leu Arg
                5                  10
TTC GAA GCG GTT CGC GAT GCG TTT GCC GCA TTG TTC GAC GGC    84
Phe Glu Ala Val Arg Asp Ala Phe Ala Ala Leu Phe Asp Gly
 15                  20                  25
ACC CAG CAA CGC GGC GCC GCA CTC TGT GTG CAG ATC GGC GGC   126
Thr Gln Gln Arg Gly Ala Ala Leu Cys Val Gln Ile Gly Gly
     30                  35                  40
GAA ACG GTG GTC GAT CTG TGG GCT GGC GTC GCC GAC AAC CAG   168
Glu Thr Val Val Asp Leu Trp Ala Gly Val Ala Asp Asn Gln
         45                  50                  55
GGC GAG CAG GCC TGG CAC AGC GAC ACG CTG GTC AAC CTG TTT   210
Gly Glu Gln Ala Trp His Ser Asp Thr Leu Val Asn Leu Phe
             60                  65                  70
TTC TGC ACC AAG ACC TTT ACC GCG GTG GCC GCG CTG CAG CTG   252
Ser Cys Thr Lys Thr Phe Thr Ala Val Ala Ala Leu Gln Leu
                 75                  80
GTC GAG GAG GGC AAG CTG GAA CTG GAT GCC CCG GTC GCG CGG   294
Val Glu Glu Gly Lys Leu Glu Leu Asp Ala Pro Val Ala Arg
 85                  90                  95
```

```
GTC TGG CCG GAA TTC GCG GCC AAT GGC AAG GAA TCG ATA ACC   336
 Val Trp Pro Glu Phe Ala Ala Asn Gly Lys Glu Ser Ile Thr
     100                 105                 110
 TTG CGC CAG CTG CTT TGT CAT CGC GCC GGG CTG CCG GCC ATT   378
 Leu Arg Gln Leu Leu Cys His Arg Ala Gly Leu Pro Ala Ile
         115                 120                 125
 CGC CGG CCG CTG GCG CCC GAG GCA CTC TAC GAC TGG ACG TGC   420
 Arg Arg Pro Leu Ala Pro Glu Ala Leu Tyr Asp Trp Thr Cys
             130                 135                 140
 ATG ACC GAC GCG CTG GCC GCC GAG GAG CCC TGG TGG GCA CCG   462
 Met Thr Asp Ala Leu Ala Ala Glu Glu Pro Trp Trp Ala Pro
                 145                 150
 GGT ACC GAC CAG GGT TAC GCC GCC ATG ACC TAC GGC TGG CTG   504
 Gly Thr Asp Gln Gly Tyr Ala Ala Met Thr Tyr Gly Trp Leu
 155                 160                 165
 GTC GGG GAG CTG CTG CGT CGT GTC GAT GGC TGC GGA GCG GGC   546
 Val Gly Glu Leu Leu Arg Arg Val Asp Gly Cys Gly Ala Gly
     170                 175                 180
 GAG TCG ATC GTT CGT CGC ACC GCG GCG CCC CTG GGG CTG GAC   588
 Glu Ser Ile Val Arg Arg Thr Ala Ala Pro Leu Gly Leu Asp
         185                 190                 195
 TTC CAT GTT GGC CTG GAC GAC AGC CAG GCC GAT CGC GTC GCT   630
 Phe His Val Gly Leu Asp Asp Ser Gln Ala Asp Arg Val Ala
             200                 205                 210
 TAC CTG ACC AGG ACC AAG AAC GAC TTC GGC GAC GCT TGC GCC   672
 Tyr Leu Thr Arg Thr Lys Asn Asp Phe Gly Asp Ala Cys Ala
                 215                 220
 CAG CGT CTG TTG AAG GCG CTG ATG AGT GAT CCT GCA TCC ATC   714
 Gln Arg Leu Leu Lys Ala Leu Met Ser Asp Pro Ala Ser Ile
 225                 230                 235
 AGC GCC CGC GCT TTC AAC AAT CCG CCA TCC ATC ATG AGC AGC   756
 Ser Ala Arg Ala Phe Asn Asn Pro Pro Ser Ile Met Ser Ser
     240                 245                 250
 GGC AAC AAG CCG GAA TGG CGA CGC ATG GCG CAG CCG GCG GCC   798
 Gly Asn Lys Pro Glu Trp Arg Arg Met Ala Gln Pro Ala Ala
         255                 260                 265
```

```
AAC GGC CAC GGC AAC GCG CGC TCG CTG GCC GGC TTG TAT ACC    840
Asn Gly His Gly Asn Ala Arg Ser Leu Ala Gly Leu Tyr Thr
            270                     275                     280
GGT TTG CTT CAG GGC AGG CTG CTC GAT GAG GCG GTC CTG CGT    882
Gly Leu Leu Gln Gly Arg Leu Leu Asp Glu Ala Val Leu Arg
                285                     290
GAG ATG ACC CAT GAG CAC AGC GCT GGC GAG GAC CGT ACC CTG    924
Glu Met Thr His Glu His Ser Ala Gly Glu Asp Arg Thr Leu
295                 300                     305
TTG GCC TCG ACG CGC TTC GGC CTG GGT TGC TGG CTC GAT CAG    966
Leu Ala Ser Thr Arg Phe Gly Leu Gly Cys Trp Leu Asp Gln
    310                     315                     320
CCT GAC GTT GCC AAT GCG ACG TTC GCC ATG GGG CCG CGC GCG   1008
Pro Asp Val Ala Asn Ala Thr Phe Ala Met Gly Pro Arg Ala
        325                     330                     335
TTC GGC CAT CCA GGT GCG GGT GGC TGC ATT GGC TTC GCC GAT   1050
Phe Gly His Pro Gly Ala Gly Gly Cys Ile Gly Phe Ala Asp
            340                     345                     350
CCG GAG CGT GAA CTG GGG TTC GGC TTC GTC ACC AAT ACT CTC   1092
Pro Glu Arg Glu Leu Gly Phe Gly Phe Val Thr Asn Thr Leu
                355                     360
GGG CCC TAT GTG CTG ATG GAC CCG AGA GCG CAA TCG CTG GCT   1134
Gly Pro Tyr Val Leu Met Asp Pro Arg Ala Gln Ser Leu Ala
365                 370                     375
CGC TGC GTA GCC GCC TGC CTG CAT TGA                       1161
Arg Cys Val Ala Ala Cys Leu His  stop codon
    380                 385
```

SEQ ID NO: 3
SEQUENCE TYPE: Nucleotide with corresponding protein
SEQUENCE LENGTH: 24 base pairs STRANDEDNESS: single
MOLECULAR TYPE: genomic DNA ORIGINAL SOURCE
ORAGANISM: Pseudomonas fluorescens
IMMEDIATE EXPERIMENTAL SOURCE
NAME OF CELL LINE: Escherichia coli PROPERTIES: aminoterminal sequence of ester hydrolase

```
GTG AGC GAC GTG CAG GTT CAG GGT TAT TTC GAT   24
Gly Ser Asp Val Gln Val Gln Gly Tyr Phe Asp
                 5                  10
```

SEQ ID NO: 4 SEQUENCE TYPE: Nucleotide with corresponding protein
SEQUENCE LENGTH: 42 base pairs STRANDEDNESS: single
TOPOLOGY: linear
MOLECULE TYPE: genomic DNA
ORIGINAL SOURCE
ORGANISM: Pseudomonas fluorescens
IMMEDIATE EXPERIMENTAL SOURCE
NAME OF CELL LINE: Escherichia coli PROPERTIES: junction sequence between Lac Z and ester hydrolase in pPF-GD3A5

```
ACC ATG ATT ACG AAT TCG AGC TCG GTA CCC TAT TTC GAT CTT   42
Thr Met Ile Thr Asn Ser Ser Ser Val Pro Tyr Phe Asp Leu
                 5                  10
```

Fig.1

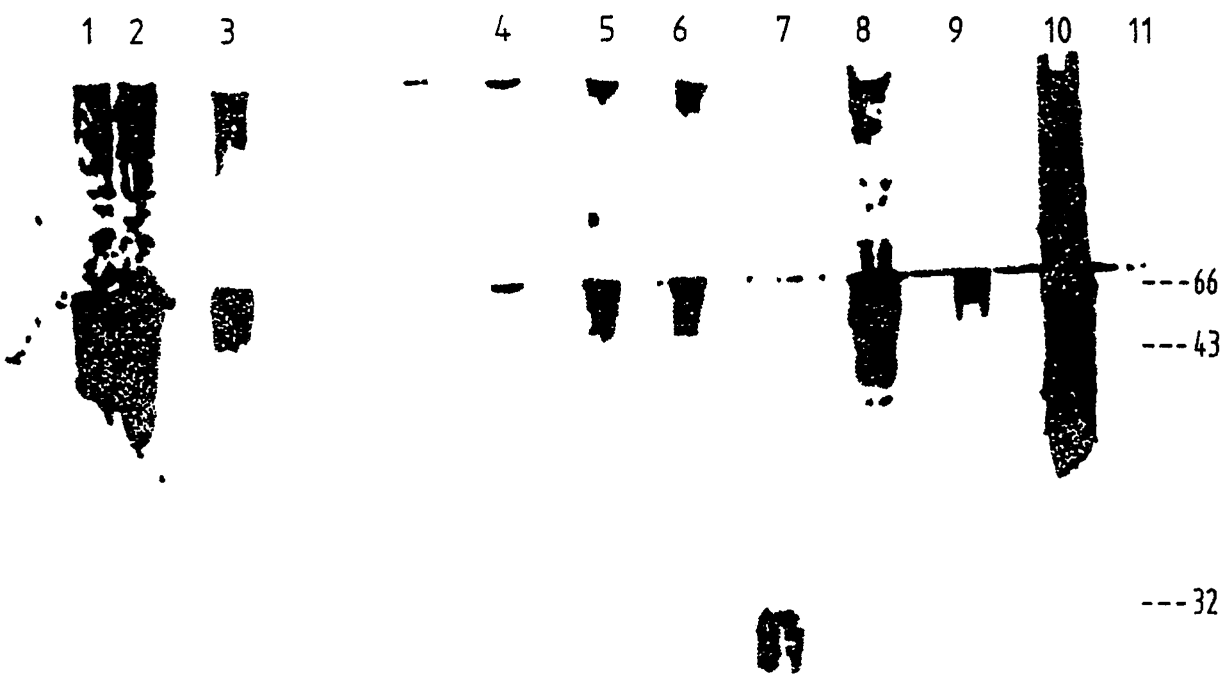


IN SITU GEL ACTIVITY ASSAY

1. SIGMA P. sp. L9518
2. SIGMA P. sp. L9518
3. AMANO P.
4. AMANO AP
5. FLUKA 425
6. AMANO AK
7. SYNTEX CLONED P. f. 3A
8. C. Cylindracea
9. Boeh. Mann. P. sp. 643327
10. FLUKA P. f.
11. PRE-STAINED MOLECULAR WEIGHT MARKERS

Fig. 2

Thermal stability of the Pseudomonas fluorescens ester hydrolase
Time,Temperature: 45,50,55

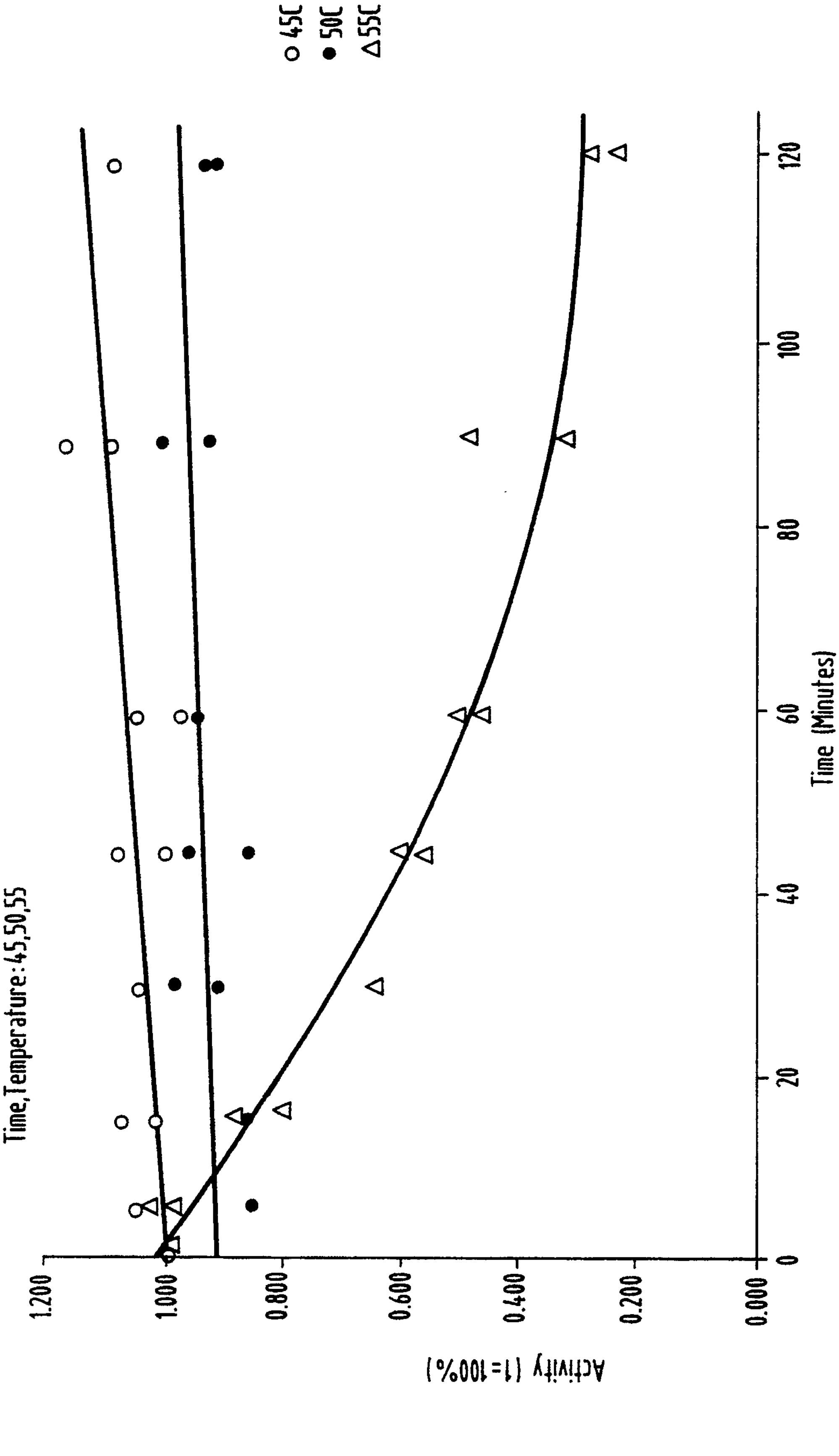

Fig. 3

Effect of Detergent and Purity on Ester Hydrolysis
50C 0.25ml Enzyme or Extract

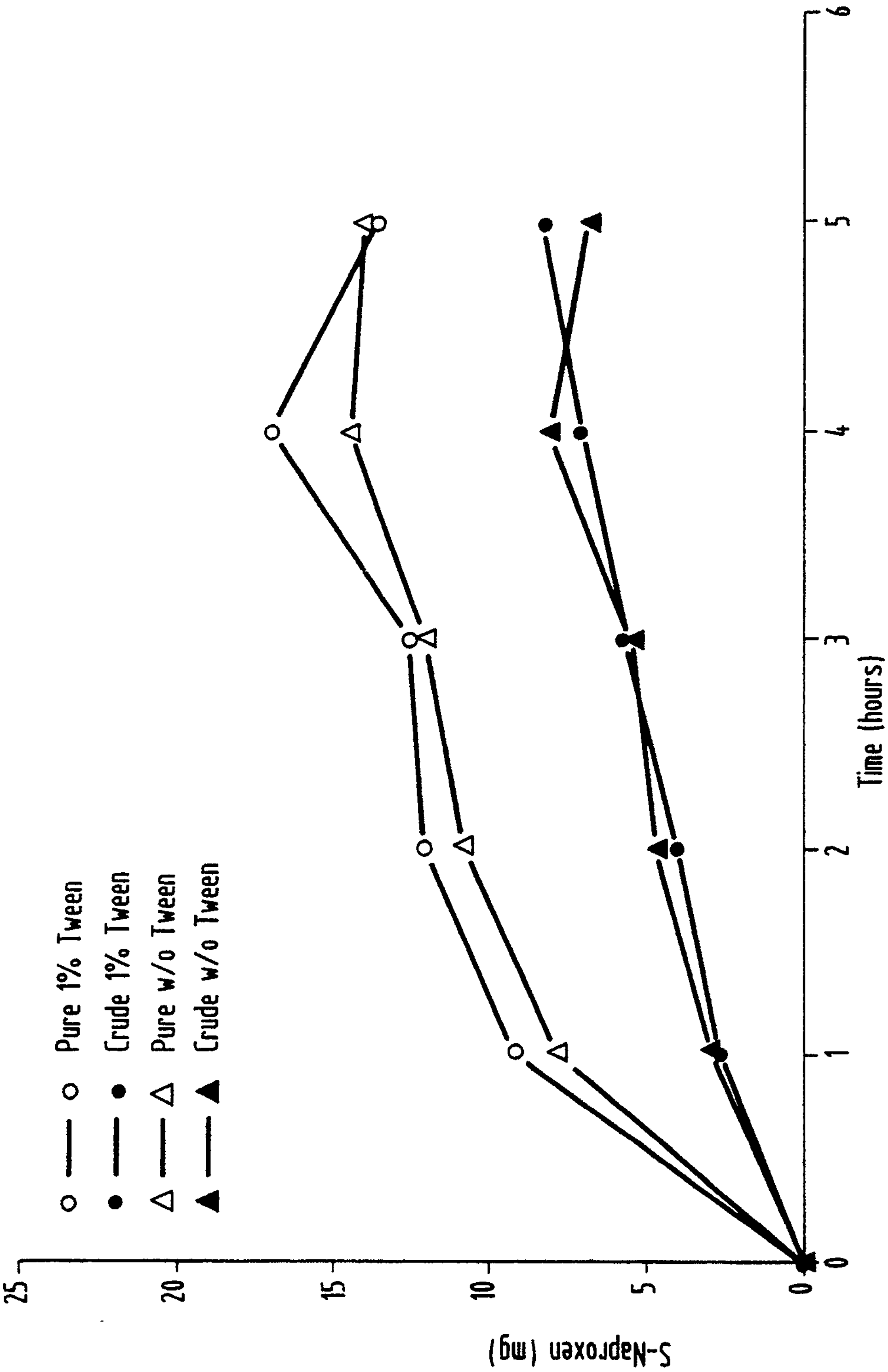

# Fig.4

Amino Terminal Sequence of the Pseudomonas fluorescens Ester Hydrolase

```
                   1          10          20
                   *           *           *
G  GGT AGC GAC GTG CAG GTT CAG GGT TAT TTC GAT.......
   GLY SER ASP VAL GLN VAL GLN GLY TYR PHE ASP
```

1. The putative ribosomal binding site is underlined.
2. The GTG at position 1-3 is translated as MET instead of VAL in E.coli.

Fig. 5

Pseudomonas fluorescens
ESTER HYDROLASE CLONE # 3A

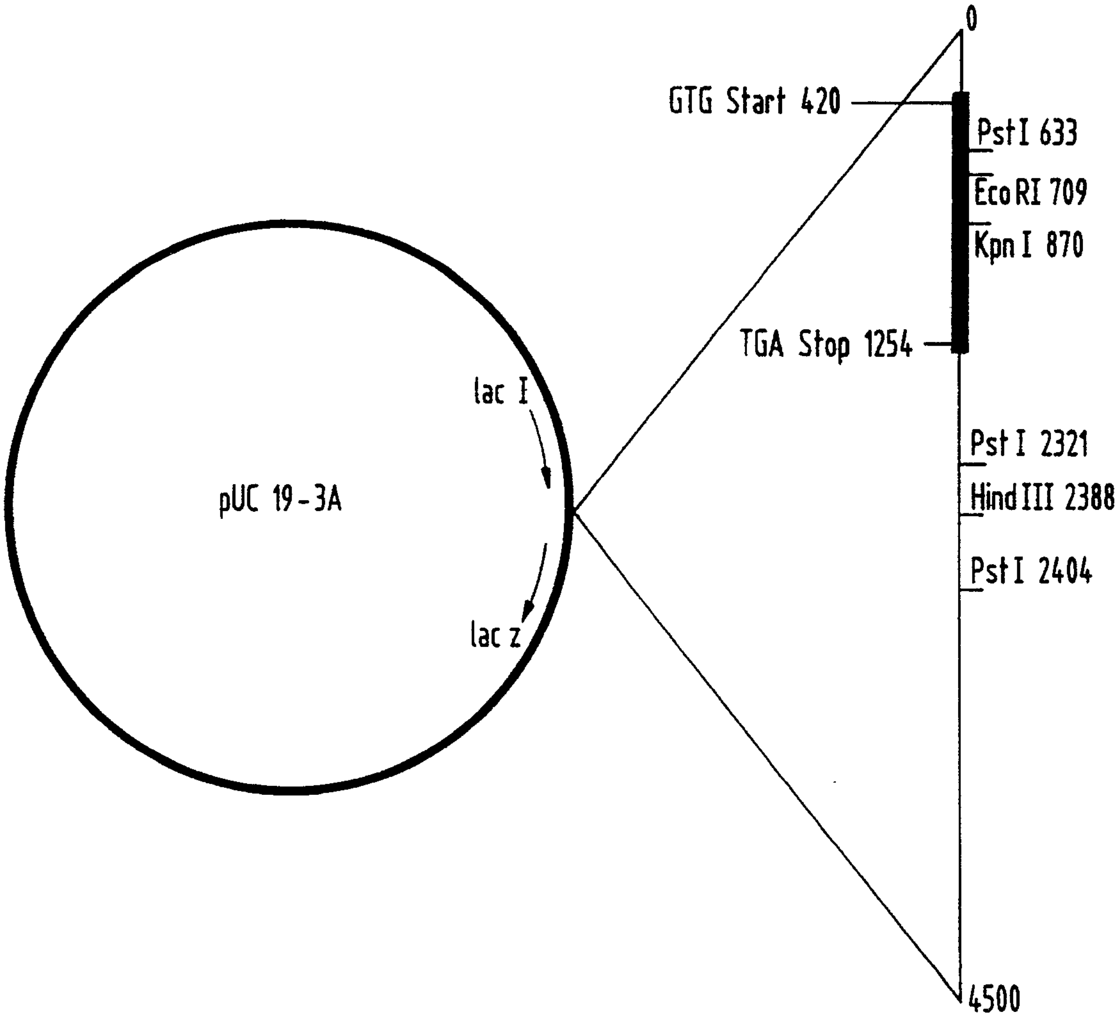

Fig.6

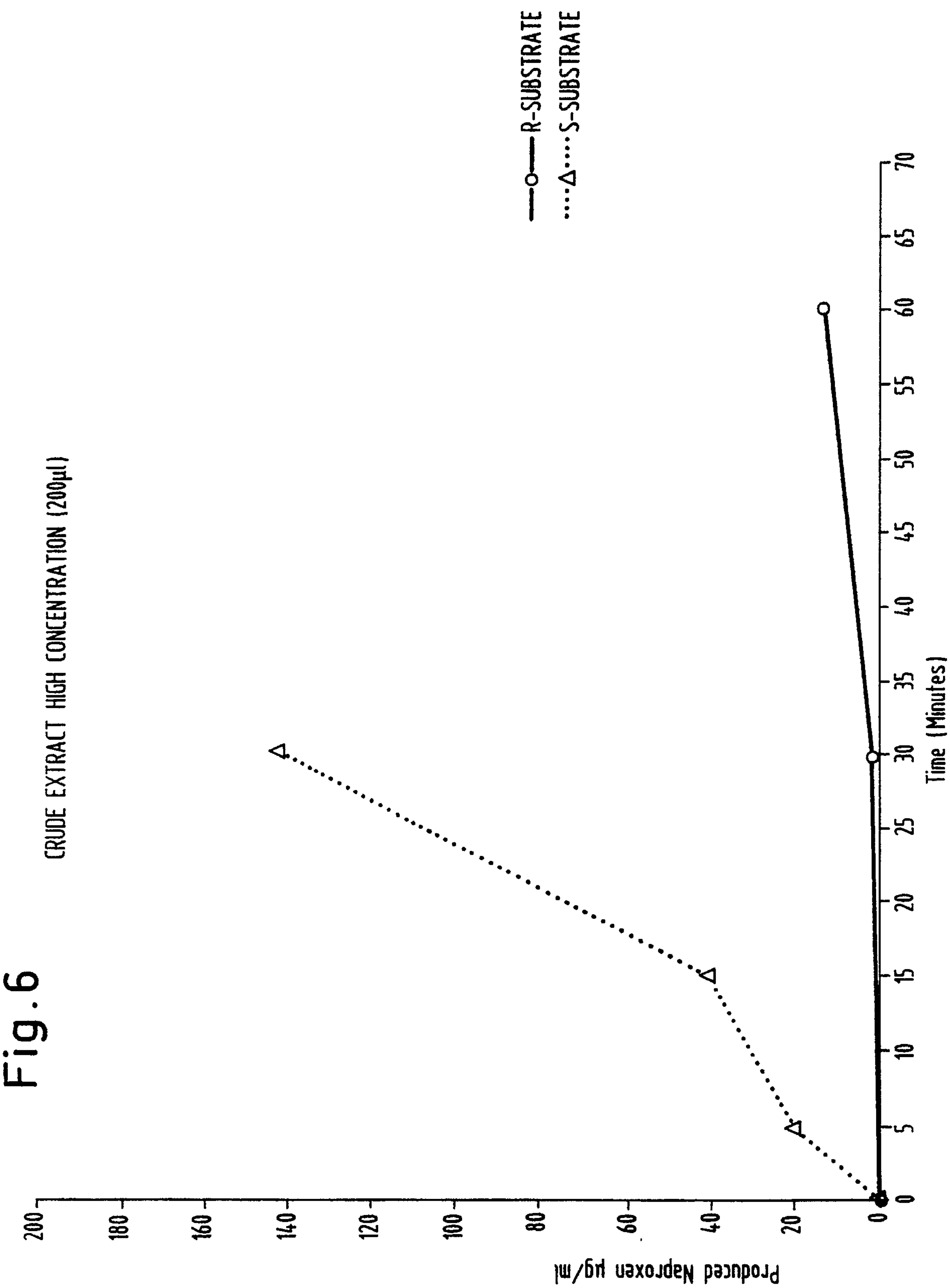
CRUDE EXTRACT HIGH CONCENTRATION (200µl)
R-SUBSTRATE
S-SUBSTRATE
Produced Naproxen µg/ml
0
20
40
60
80
100
120
140
160
180
200
Time (Minutes)
0
5
10
15
20
25
30
35
40
45
50
55
60
65
70

# Fig.7

Junction Sequence between Lac Z and the Ester Hydrolase in pPF-GD3A5

```
ATG ACC ATG ATT ACG AAT TCG AGC TCG GTA CCC TAT TTC GAT CTT
    THR MET ILE THR ASN SER SER SER VAL PRO TYR PHE ASP LEU
```

Underlined sequence represents the cloned ester hydrolase sequence.

# Fig. 8

Thermal stability of the mutants

Thermal Stability of Esterase Mutants at 60C

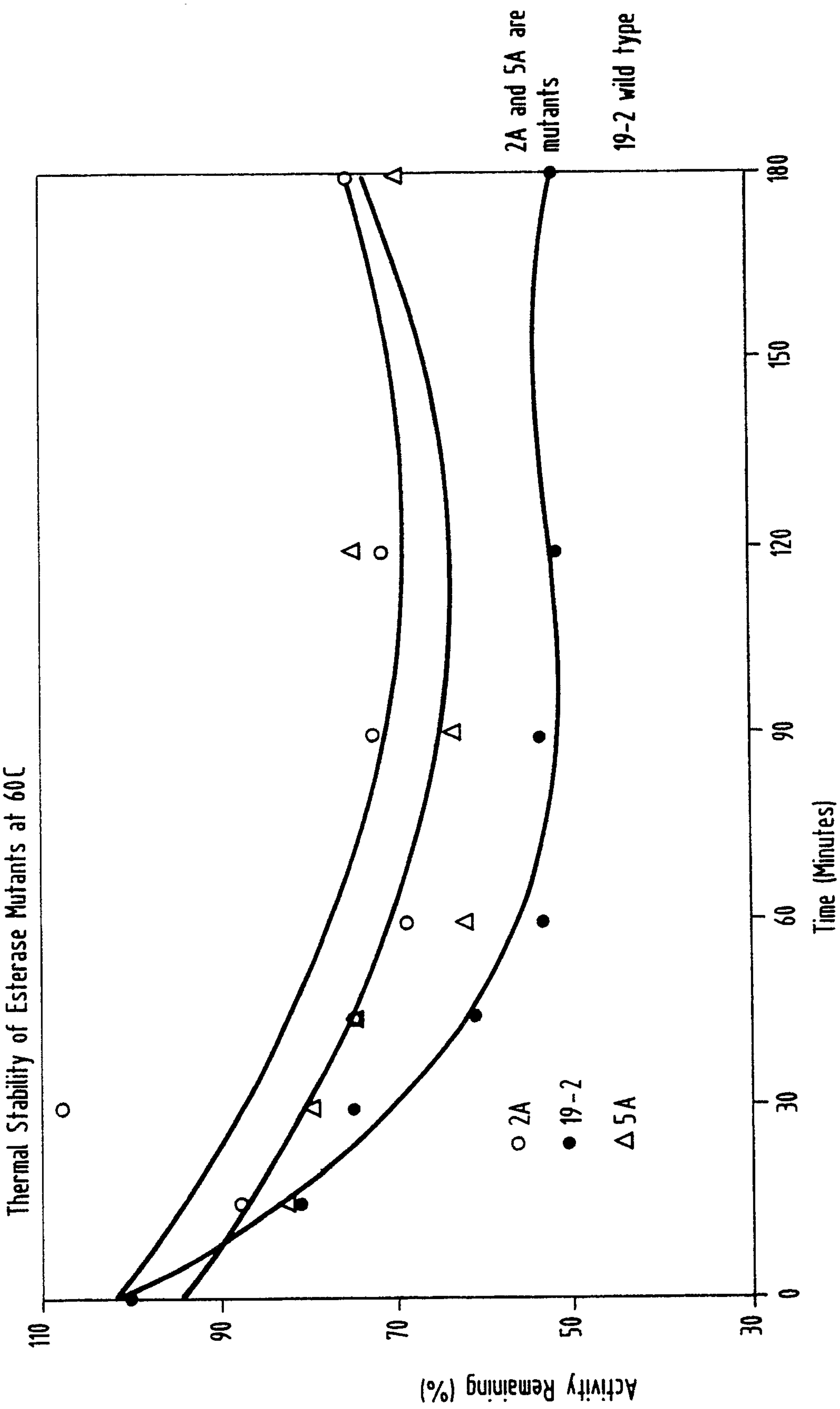

# Fig.9

Comparison of N-terminal sequence of authentic ester hydrolase
from pPF-3A and fused ester hydrolase from pPF-GD3A

authentic ester hydrolase:

```
      G GGT AGC GAC GTG CAG GTT CAG GGT TAT TTC GAT CTT
        GLY SER ASP VAL GLN VAL GLN GLY TYR PHE ASP LEU
                    ***
```

Fused ester hydrolase:

```
ATG ACC ATG ATT ACG AAT TCG AGC TCG GTA CCC TAT TTC GAT CTT
    THR MET ILE THR ASN SER SER SER VAL PRO TYR PHE ASP LEU
```

1. Underlined sequence represents the cloned ester hydrolase sequence.
2. Portion of sequence in bold represents the *lac* Z portion.
3. (***) Asterisk indicates start codon for expression of authentic ester hydrolase (GTG is translated as MET instead of VAL in *E.coli*).

FIG. 10 A

Met

Val Gln Val Gln Gly Tyr Phe Asp Leu Arg Phe Glu Ala Val Arg Asp Ala

GTG CAG GTT CAG GGT TAT TTC GAT CTT CGC TTC GAA GCG GTT CGC GAT GCG

1 9 18 27 36 45

Phe Ala Ala Leu Phe Asp Gly Thr Gln Gln Arg Gly Ala Ala Leu Cys Val Gln Ile

TTT GCC GCA TTG TTC GAC GGC ACC CAG CAA CGC GGC GCC GCA CTC TGT GTG CAG ATC

60 69 78 87 96 105

Gly Gly Glu Thr Val Val Asp Leu Trp Ala Gly Val Ala Asp Asn Gln Gly Glu Gln

GGC GGC GAA ACG GTG GTC GAT CTG TGG GCT GGC GTC GCC GAC AAC CAG GGC GAG CAG

117 126 135 144 153 162

Ala Trp His Ser Asp Thr Leu Val Asn Leu Phe Ser Cys Thr Lys Thr Phe Thr Ala

GCC TGG CAC.AGC GAC ACG CTG GTC AAC CTG TTT TCC TGC ACC AAG ACC TTT ACC GCG

174 183 192 201 210 219

Val Ala Ala Leu Gln Leu Val Glu Glu Gly Lys Leu Glu Leu Asp Ala Pro Val Ala

GTG GCC GCG CTG CAG CTG GTC GAG GAG GGC AAG CTG GAA CTG GAT GCC CCG GTC GCG

231 240 249 258 267 276

Arg Val Trp Pro Glu Phe Ala Ala Asn Gly Lys Glu Ser Ile Thr Leu Arg Gln Leu

CGG GTC TGG CCG GAA TTC GCG GCC AAT GGC AAG GAA TCG ATA ACC TTG CGC CAG CTG

288 297 306 315 324 333

Leu Cys His Arg Ala Gly Leu Pro Ala Ile Arg Arg Pro Leu Ala Pro Glu Ala Leu

CTT TGT CAT CGC GCC GGG CTG CCG GCC ATT CGC CGG CCG CTG GCG CCC GAG GCA CTC

345 354 363 372 381 390

Tyr Asp Trp Thr Cys Met Thr Asp Ala Leu Ala Ala Glu Glu Pro Trp Trp Ala Pro

TAC GAC TGG ACG TGC ATG ACC GAC GCG CTG GCC GCC GAG GAG CCC TGG TGG GCA CCG

402 411 420 429 438 447

Gly Thr Asp Gln Gly Tyr Ala Ala Met Thr Tyr Gly Trp Leu Val Gly Glu Leu Leu

GGT ACC GAC CAG GGT TAC GCC GCC ATG ACC TAC GGC TGG CTG GTC GGG GAG CTG CTG

459 468 477 486 495 504

Arg Arg Val Asp Gly Cys Gly Ala Gly Glu Ser Ile Val Arg Arg Thr Ala Ala Pro

CGT CGT GTC GAT GGC TGC GGA GCG GGC GAG TCG ATC GTT CGT CGC ACC GCG GCG CCC

516 525 534 543 552 561

FIG. 10 B

Leu Gly Leu Asp Phe His Val Gly Leu Asp Asp Ser Gln Ala Asp Arg Val Ala Tyr
CTG GGG CTG GAC TTC CAT GTT GGC CTG GAC GAC AGC CAG GCC GAT CGC GTC GCT TAC
573 582 591 600 609 618

Leu Thr Arg Thr Lys Asn Asp Phe Gly Asp Ala Cys Ala Gln Arg Leu Leu Lys Ala
CTG ACC AGG ACC AAG AAC GAC TTC GGC GAC GCT TGC GCC CAG CGT CTG TTG AAG GCG
630 639 648 657 666 675

Leu Met Ser Asp Pro Ala Ser Ile Ser Ala Arg Ala Phe Asn Asn Pro Pro Ser Ile
CTG ATG AGT GAT CCT GCA TCC ATC AGC GCC CGC GCT TTC AAC AAT CCG CCA TCC ATC
687 696 705 714 723 732

Met Ser Ser Gly Asn Lys Pro Glu Trp Arg Arg Met Ala Gln Pro Ala Ala Asn Gly
ATG AGC AGC GGC AAC AAG CCG GAA TGG CGA CGC ATG GCG CAG CCG GCG GCC AAC GGC
744 753 762 771 780 789

His Gly Asn Ala Arg Ser Leu Ala Gly Leu Tyr Thr Gly Leu Leu Gln Gly Arg Leu
CAC GGC AAC GCG CGC TCG CTG GCC GGC TTG TAT ACC GGT TTG CTT CAG GGC AGG CTG
801 810 819 828 837 846

Leu Asp Glu Ala Val Leu Arg Glu Met Thr His Glu His Ser Ala Gly Glu Asp Arg
CTC GAT GAG GCG GTC CTG CGT GAG ATG ACC CAT GAG CAC AGC GCT GGC GAG GAC CGT
858 867 876 885 894 903

Thr Leu Leu Ala Ser Thr Arg Phe Gly Leu Gly Cys Trp Leu Asp Gln Pro Asp Val
ACC CTG TTG GCC TCG ACG CGC TTC GGC CTG GGT TGC TGG CTC GAT CAG CCT GAC GTT
915 924 933 942 951 960

Ala Asn Ala Thr Phe Ala Met Gly Pro Arg Ala Phe Gly His Pro Gly Ala Gly Gly
GCC AAT GCG ACG TTC GCC ATG GGG CCG CGC GCG TTC GGC CAT CCA GGT GCG GGT GGC
972 981 990 999 1008 1017

Cys Ile Gly Phe Ala Asp Pro Glu Arg Glu Leu Gly Phe Gly Phe Val Thr Asn Thr
TGC ATT GGC TTC GCC GAT CCG GAG CGT GAA CTG GGG TTC GGC TTC GTC ACC AAT ACT
1029 1038 1047 1056 1065 1074

Leu Gly Pro Tyr Val Leu Met Asp Pro Arg Ala Gln Ser Leu Ala Arg Cys Val Ala
CTC GGG CCC TAT GTG CTG ATG GAC CCG AGA GCG CAA TCG CTG GCT CGC TGC GTA GCC
1086 1095 1104 1113 1122 1131

Ala Cys Leu His TER
GCC TGC CTG CAT TGA
1143

# Fig.11A

```
ATG ATT ACG AAT TCG AGC TCG GTA CCC TAT TTC GAT CTT CGC   42
Met Ile Thr Asn Ser Ser Ser Val Pro Tyr Phe Asp Leu Arg
                 5                  10
TTC GAA GCG GTT CGC GAT GCG TTT GCC GCA TTG TTC GAC GGC   84
Phe Glu Ala Val Arg Asp Ala Phe Ala Ala Leu Phe Asp Gly
 15                 20                  25
ACC CAG CAA CGC GGC GCC GCA CTC TGT GTG CAG ATC GGC GGC  126
Thr Gln Gln Arg Gly Ala Ala Leu Cys Val Gln Ile Gly Gly
     30                  35                  40
GAA ACG GTG GTC GAT CTG TGG GCT GGC GTC GCC GAC AAC CAG  168
Glu Thr Val Val Asp Leu Trp Ala Gly Val Ala Asp Asn Gln
         45                  50                  55
GGC GAG CAG GCC TGG CAC AGC GAC ACG CTG GTC AAC CTG TTT  210
Gly Glu Gln Ala Trp His Ser Asp Thr Leu Val Asn Leu Phe
             60                  65                  70
TTC TGC ACC AAG ACC TTT ACC GCG GTG GCC GCG CTG CAG CTG  252
Ser Cys Thr Lys Thr Phe Thr Ala Val Ala Ala Leu Gln Leu
                 75                  80
GTC GAG GAG GGC AAG CTG GAA CTG GAT GCC CCG GTC GCG CGG  294
Val Glu Glu Gly Lys Leu Glu Leu Asp Ala Pro Val Ala Arg
 85                 90                  95
GTC TGG CCG GAA TTC GCG GCC AAT GGC AAG GAA TCG ATA ACC  336
Val Trp Pro Glu Phe Ala Ala Asn Gly Lys Glu Ser Ile Thr
    100                 105                 110
TTG CGC CAG CTG CTT TGT CAT CGC GCC GGG CTG CCG GCC ATT  378
Leu Arg Gln Leu Leu Cys His Arg Ala Gly Leu Pro Ala Ile
        115                 120                 125
CGC CGG CCG CTG GCG CCC GAG GCA CTC TAC GAC TGG ACG TGC  420
Arg Arg Pro Leu Ala Pro Glu Ala Leu Tyr Asp Trp Thr Cys
            130                 135                 140
ATG ACC GAC GCG CTG GCC GCC GAG GAG CCC TGG TGG GCA CCG  462
Met Thr Asp Ala Leu Ala Ala Glu Glu Pro Trp Trp Ala Pro
                145                 150
GGT ACC GAC CAG GGT TAC GCC GCC ATG ACC TAC GGC TGG CTG  504
Gly Thr Asp Gln Gly Tyr Ala Ala Met Thr Tyr Gly Trp Leu
155                 160                 165
```

# Fig.11B

```
GTC GGG GAG CTG CTG CGT CGT GTC GAT GGC TGC GGA GCG GGC   546
Val Gly Glu Leu Leu Arg Arg Val Asp Gly Cys Gly Ala Gly
    170                 175                 180
GAG TCG ATC GTT CGT CGC ACC GCG GCG CCC CTG GGG CTG GAC   588
Glu Ser Ile Val Arg Arg Thr Ala Ala Pro Leu Gly Leu Asp
        185                 190                 195
TTC CAT GTT GGC CTG GAC GAC AGC CAG GCC GAT CGC GTC GCT   630
Phe His Val Gly Leu Asp Asp Ser Gln Ala Asp Arg Val Ala
            200                 205                 210
TAC CTG ACC AGG ACC AAG AAC GAC TTC GGC GAC GCT TGC GCC   672
Tyr Leu Thr Arg Thr Lys Asn Asp Phe Gly Asp Ala Cys Ala
                215                 220
CAG CGT CTG TTG AAG GCG CTG ATG AGT GAT CCT GCA TCC ATC   714
Gln Arg Leu Leu Lys Ala Leu Met Ser Asp Pro Ala Ser Ile
225                 230                 235
AGC GCC CGC GCT TTC AAC AAT CCG CCA TCC ATC ATG AGC AGC   756
Ser Ala Arg Ala Phe Asn Asn Pro Pro Ser Ile Met Ser Ser
    240                 245                 250
GGC AAC AAG CCG GAA TGG CGA CGC ATG GCG CAG CCG GCG GCC   798
Gly Asn Lys Pro Glu Trp Arg Arg Met Ala Gln Pro Ala Ala
        255                 260                 265
AAC GGC CAC GGC AAC GCG CGC TCG CTG GCC GGC TTG TAT ACC   840
Asn Gly His Gly Asn Ala Arg Ser Leu Ala Gly Leu Tyr Thr
            270                 275                 280
GGT TTG CTT CAG GGC AGG CTG CTC GAT GAG GCG GTC CTG CGT   882
Gly Leu Leu Gln Gly Arg Leu Leu Asp Glu Ala Val Leu Arg
                285                 290
GAG ATG ACC CAT GAG CAC AGC GCT GGC GAG GAC CGT ACC CTG   924
Glu Met Thr His Glu His Ser Ala Gly Glu Asp Arg Thr Leu
295                 300                 305
TTG GCC TCG ACG CGC TTC GGC CTG GGT TGC TGG CTC GAT CAG   966
Leu Ala Ser Thr Arg Phe Gly Leu Gly Cys Trp Leu Asp Gln
    310                 315                 320
CCT GAC GTT GCC AAT GCG ACG TTC GCC ATG GGG CCG CGC GCG  1008
Pro Asp Val Ala Asn Ala Thr Phe Ala Met Gly Pro Arg Ala
        325                 330                 335
```

# Fig.11C

```
TTC GGC CAT CCA GGT GCG GGT GGC TGC ATT GGC TTC GCC GAT  1050
Phe Gly His Pro Gly Ala Gly Gly Cys Ile Gly Phe Ala Asp
            340                 345                 350
CCG GAG CGT GAA CTG GGG TTC GGC TTC GTC ACC AAT ACT CTC  1092
Pro Glu Arg Glu Leu Gly Phe Gly Phe Val Thr Asn Thr Leu
                355                 360
GGG CCC TAT GTG CTG ATG GAC CCG AGA GCG CAA TCG CTG GCT  1134
Gly Pro Tyr Val Leu Met Asp Pro Arg Ala Gln Ser Leu Ala
365                 370                 375
CGC TGC GTA GCC GCC TGC CTG CAT TGA                      1161
Arg Cys Val Ala Ala Cys Leu His  stop codon
    380                 385
```